(19) Europäisches Patentamt
European Patent Office
Office européen des brevets

(11) **EP 3 813 542 B1**

(12) **EUROPEAN PATENT SPECIFICATION**

(45) Date of publication and mention
of the grant of the patent:
**27.05.2026 Bulletin 2026/22**

(21) Application number: **19732700.0**

(22) Date of filing: **27.06.2019**

(51) International Patent Classification (IPC):
*A23K 10/16* (2016.01)    *A23L 33/135* (2016.01)
*A61K 35/741* (2015.01)   *A61K 9/14* (2006.01)
*A61K 9/20* (2006.01)     *A61K 39/00* (2006.01)
*A61K 35/66* (2015.01)

(52) Cooperative Patent Classification (CPC):
**A61K 9/14; A23K 10/16; A23L 33/135; A61K 9/20;
A61K 35/66;** A23K 30/00; A23K 50/10; A23K 50/70;
A23K 50/80; A23V 2200/3204; A23V 2200/324;
A61K 2039/552; Y02A 40/818

(86) International application number:
**PCT/EP2019/067236**

(87) International publication number:
**WO 2020/002543 (02.01.2020 Gazette 2020/01)**

(54) **METHOD FOR PREPARING A COMPOSITION COMPRISING ATTENUATED OR KILLED METHANOBREVIBACTER ARCHAEBACTERIA CELLS AND COMPOSITION THEREBY OBTAINED**

VERFAHREN ZUR HERSTELLUNG EINER ZUSAMMENSETZUNG MIT ABGESCHWÄCHTEN ODER ABGETÖTETEN METHANO-BREVIBACTER-ARCHAEBAKTERIEN-ZELLEN UND DADURCH ERHALTENE ZUSAMMENSETZUNG

PROCÉDÉ DE PRÉPARATION D'UNE COMPOSITION COMPRENANT DES CELLULES D'ARCHÉOBACTÉRIES MÉTHANOBREVIBACTER METHANOBREVIBACTER ATTÉNUÉES OU TUÉES ET COMPOSITION AINSI OBTENUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.06.2018 EP 18180236**

(43) Date of publication of application:
**05.05.2021 Bulletin 2021/18**

(73) Proprietor: **Arkaiya SA
1015 Lausanne (CH)**

(72) Inventor: **Sutherland, Duncan-Bruce
1027 Lonay (CH)**

(74) Representative: **Schneiter, Sorin et al
ABREMA SA
Avenue du Théâtre 16
1005 Lausanne (CH)**

(56) References cited:
**US-A1- 2012 034 198    US-A1- 2016 074 440**

• **BURGUE�O JOAN F. ET AL: "TLR2 and TLR9 modulate enteric nervous system inflammatory responses to lipopolysaccharide", JOURNAL OF NEUROINFLAMMATION, vol. 13, no. 1, 18 August 2016 (2016-08-18), GB, XP093278452, ISSN: 1742-2094, DOI: 10.1186/s12974-016-0653-0**
• **M MILLION ET AL: "Obesity-associated gut microbiota is enriched in Lactobacillus reuteri and depleted in Bifidobacterium animalis and Methanobrevibacter smithii", INTERNATIONAL JOURNAL OF OBESITY., vol. 36, no. 6, 1 June 2012 (2012-06-01), GB, pages 817 - 825, XP055622556, ISSN: 0307-0565, DOI: 10.1038/ijo.2011.153**

**(Cont. next page)**

- **CORINNA BANG ET AL: "The Intestinal Archaea Methanosphaera stadtmanae and Methanobrevibacter smithii Activate Human Dendritic Cells", PLOS ONE, vol. 9, no. 6, E99411, 10 June 2014 (2014-06-10), pages 1 - 8, XP055590654, DOI: 10.1371/ journal.pone.0099411**

**Description**

**FIELD OF THE INVENTION**

The invention relates to a process for preparing a composition comprising attenuated or killed *methanobrevibacter* Archaeabacteria cells for incorporation at high dosage in food/feed/tablets. Also disclosed are compositions obtained by the process of the invention for use in a method for reducing immune pathology and disease severity caused by infections as well as in a method for improving the resistance to intestinal pathogen infection and/or for reducing intestinal inflammation in a subject.

**BACKGROUND OF THE INVENTION**

Probiotics are live microorganisms that when taken as dietary adjunct confer health benefits to the recipient that include improved immune system and metabolic functions. Dense symbiotic communities of microorganisms that support digestion and immunological fitness colonize the vertebrate gut. Throughout evolution, symbiotic micro-organisms are known to co-localize within their micro-environment, collectively referred to as the microbiota; for instance, in ruminants (e.g. cows), micro-organisms promoting fibre digestion in the gut including yeast, cellulolytic bacteria and archaea, co-localize in the rumen. Archaea probiotics supplemented in feed are adept at supporting the microbiota functions.

For example, WO 2016/147121 A1 (ECOLE POLYTECHNIQUE FEDERALE DE LAUSANNE) discloses a food supplement comprising or consisting of *Archaebacteria,* and particularly methanogenic *Archaebacteria,* to be used as a probiotic adjunct for animal feed. The supplement can be provided to e.g. farmed animals in addition to standard feed or as a food composition. Such a supplement is particularly useful in aquaculture and proves able in increasing animal growth rates, reducing animal susceptibility to parasitic infections and/or ameliorating animal faecal waste impact on environment. Also encompassed are methods of manufacturing a composition comprising the bioactive food supplement as well as uses thereof.

Methanobrevibacter (Mbb) archaea constitute an important fraction of the intestinal microbiota for many herbivorous vertebrates. In healthy humans, Methanobrevibacter smithii account for approximately 10% of the anaerobic microbiota resident in the large intestine. Mbb metabolize the hydrogen produced by cellulolytic bacteria to produce methane gas generating a "hydrogen sink" necessary for the cellulose digesting microbiota to thrive. Methane producing Mbb thus play a critical role in the dietary metabolism of herbivorous animals (notably in ruminants).

It is well established that the symbiotic intestinal microbiota is tolerated by the immune system of healthy hosts. The symbiotic microbiota promotes intestinal immune homeostasis. This is in contrast to pathogenic microbes that induce inflammation and pathology.

Since Mbb is a major constituent of the herbivore intestinal microbiota, Mbb and the host immune system have likely co-adapted through evolution in a way so as to promote immune tolerance and intestinal homeostasis. WO 2016/147121 A1 previously demonstrated beneficial immune modulating properties of Mbb. The use of Mbb as probiotic or food/feed supplement can thus improve immune system function.

EP 2 251 017 A1 (CEDARS SINAI MEDICAL CENTER) and US 6,328,959 B1 (Kayar S. et a.) describe the benefit of methane production in certain situations, in particular they report about methane production using methanogen delivery.

US 2012/034198 A1 (Garner et al.) describes processes and formulations for the administration and storage of dried microorganisms. Microbial based products containing prebiotic formulations for administration and for storage of microorganisms are disclosed. These prebiotic formulations can act both for administration into an animal or human subject and act as to preserve or store the dried microorganisms. In particular, methods for conservation of freeze-dried Lactic acid bacteria during storage at ambient temperature and at 37 °C are described. Lactobacillus species are widely used as prebiotics because they can favor the health and well-being of humans and the health and productivity of animals by inhibiting the growth of pathogenic bacteria and by enhancing gastrointestinal immune function. Probiotic compounds can be administered together with prebiotic bacteria and can stimulate the colonization of the gastrointestinal tract by prebiotic bacteria because they are preferably metabolized by prebiotic bacteria. For the use of Lactobacillus species as prebiotics important steps are concentration of the bacteria e.g. by centrifugation and freeze drying or storage at low temperatures. For the practical use as prebiotics involving shipping, shelf storage etc., it is important that the micro-organisms retain viability during incubation at room temperature and at temperatures up to 37 °C and by adding fructooligosaccharides as probiotic and calcium stearate as flow agent to freeze dried bacteria. The authors have shown in experiments with several Lactobacillus species that the addition of these components increases the viability of these bacteria during incubation at higher temperatures. Lactobacillus species are fermentative moderate anaerobes but can be handled and plated without specific equipment like anaerobic chambers and gassing manifolds because their tolerance against oxygen is much higher than that of methanogens which are extremely sensitive to small amounts of oxygen in axenic cultures. Therefore it cannot be anticipated that freeze drying of methanogens using standard conditions will work as a method of conservation of methanogens. Lyophilization in horse serum supplemented with 5 percent glucose and 3mg/ml of ferrous sulfide followed by storage at either 8 or -70 °C has been reported as a method for conservation of some species of Methanobacteriaceae but did not work well for Methanococcus and Methanospirillum (reviewed by Whitman et al., in The Prokaryotes, Rosenberg et al. eds. 2014,pp.123-163, Springer Verlag Berlin Heidelberg).

US 2016/074440 A1 (Brugere et al.) discloses a composition containing a microorganism, preferably an Archaea, expressing a TMA methyltransferase and a TMA methyl group acceptor corrinoid protein, capable of metabolizing trimethylamine (TMA) in the presence of hydrogen in a human cavity, such as the intestine or the vagina, for use as a medicament for treating, reducing or eliminating TMA at the level of the human cavity. In addition, a composition containing a TMA methyltransferase and a TMA methyl group acceptor corrinoid protein. These compositions are of use for treating trimethylaminuria, for treating vaginal fluids in the case of bacterial vaginosis and for reducing or eliminating odours due to TMA. These compositions are also of use for reducing the level of plasma TMAO, for preventing the formation of atheroma plaques and/or for preventing cardiovascular diseases. In particular, Brugere et al. show that the metabolic functions of live Mbb archaea (notably TMA methyltransferase capable of metabolizing trimethylamine (TMA)) can be harnessed to treat vaginosis and other medical conditions that require TMA to be metabolized for their treatment.

Corinna Bang et al. "The Intestinal Archaea Methanosphaera stadtmanae and Methanobrevibacter smithii Activate Human Dendritic Cells" PLOS ONE Vol. 9, no 6, E99411, 10 June 2014 (XP055590654) show that live archaea *M. stadtmanae* and *M. smithii* can stimulate TNF-a and IL-1b secretion in human dendritic cells (20 hour stimulation of $2 \times 10^5$ moDCs followed by an ELISA detection method). The archaea cells in the study are living cells that have not been inactivated. The study does not demonstrate a capacity for Mbb archaea cells to inhibit inflammatory responses.

A potential negative side effect of using Mbb as live probiotic or food/feed supplement is the excessive generation of methane gas. Excessive flatulence or accumulation of methane in the gastro-intestinal tract is not a desired effect for animal or human consumers. For example, Uday C Ghoshal et al. "Slow Transit Constipation Associated with Excess Methane Production and Its Improvement Following Rifaximin Therapy: A Case Report" in J. Neurogastroenterol Motil. Published on 2011 Apr. 27: https://www.ncbi.nlm.nih.gov/pmc/articles/PMC3093012/ and Attaluri A. et al. "Methanogenic flora is associated with altered colonic transit but not stool characteristics in constipation without IBS" Am. J. Gastroenterol. 2010 June; 105(6):1407-11 https://www.ncbi.nlm.nih.gov/pubmed/19953090 highlighting the drawback of excessive methane in relation to bowel movements.

In the case of agriculture, methane is a problematic greenhouse gas contributing to global warming. Thus, an important drawback of using Mbb to support immune system function is the unwanted excessive methane production.

Live intestinal archaea when used as a probiotic supplement at $10^5$-$10^8$ per gram of composition in animal feed is shown to improve growth rates and improve immune system function.

The immune stimulating properties of archaea probiotic supplements are dose responsive; however, the inefficient cultivation of high microbial biomass limits the use of high dosages for application in feed and food. A more efficient means to enrich intestinal archaea for the food and feed industry remains an important innovation gap. Secondly, ensuring the viability of the probiotic in processed food/feed provides a challenge for quality control and certification processes. Therefore a way to increase the immune stimulant potency as well as improving the ease of quality control remain key barriers of archaea based supplements for human and animal consumption in food/feed.

There is a need for a technical solution that enables the immune modulating benefits of Mbb without increasing methane by-products in the recipients. Increasing the immune-modulating potency such as the resistance to intestinal pathogen infection or the reduction of intestinal inflammation in a subject is also desirable. It is thus an object of the invention among others to overcome those technical problems.

## BRIEF DESCRIPTION OF THE INVENTION

Interestingly, Applicants have observed the immune-modulating characteristics of intestinal archaea do not depend uniquely on the viability of the intestinal archaea; instead, Applicants observed that the microbe-associated-molecular-patterns (MAMPS) present in non-live *methanobrevibacter* (Mbb) archaea are also found to be immunogenic when incorporated in food and feed. While intestinal archaea are strictly anaerobic and do not grow in the presence of oxygen, some strains are reported to be aero-tolerant. Archaea have adapted distinct cell features enabling them to survive under extreme conditions. Therefore the treatment conditions needed to inactivate intestinal *methanobrevibacter* archaea remain unclear. A method to inactivate *methanobrevibacter* archaea while preserving the immunogenic MAMPS will facilitate the quality control needed to implement archaea based supplements for human and animal consumption in food/feed. Besides, it is submitted that the prior art never explored the inactivation thresholds of Mbb archaea.

Here Applicants describe how to inactivate the resulting concentrated biomass using a combination of air, heat or infra-red irradiation exposure in a way that preserves the MAMPS. When the enriched inactivated archaea formulation is included in feed at a ratio equivalent of $10^5$-$10^{12}$ archaea cells per gram feed composition, experimental animals were significantly more resistant against immune pathology, disease and mortality induced by dietary or infectious challenges.

One object of the present invention is directed to a process for preparing a composition comprising attenuated or killed *methanobrevibacter Archaebacteria* cells for incorporation in human food/tablets and/or animal feed/tablets, the process comprising:

a) collecting a liquid suspension of *methanobrevibacter Archaebacteria* cells;

b) attenuating or killing said *methanobrevibacter Archaebacteria* cells in said liquid suspension;

c) freeze drying said liquid suspension to obtain an attenuated or killed *methanobrevibacter Archaebacteria* cell composition, characterized in that said attenuated of killed *methanobrevibacter Archaebacteria* cells have preserved at least 80% of the microbe-associated-molecular-patterns (MAMPS) signal potential through the host pattern recognition receptor system, wherein said MAPMS signal potential is determined by the RNA expression level of NF-κB in *in vitro* cell cultures.

Another object of the invention is to provide a composition comprising attenuated or killed *methanobrevibacter Archaebacteria* cells obtained according to the process of the invention, wherein said attenuated or killed *methanobrevibacter Archaebacteria* cells have preserved at least 80% of the microbe-associated-molecular-patterns (MAMPS) signal potential through the host pattern recognition receptor system and wherein said composition comprises at least $10^8$ attenuated or killed *methanobrevibacter Archaebacteria* cells per gram of said composition, for use in method for improving the resistance to intestinal pathogen infection and/or for reducing intestinal inflammation in a subject.

In embodiments, the present invention provides a composition comprising attenuated or killed *methanobrevibacter Archaebacteria* cells obtained according to the process of the invention, wherein said inactivated or killed *methanobrevibacter Archaebacteria* cells have preserved at least 80% of the microbe-associated-molecular-patterns (MAMPS) signal potential through the host pattern recognition receptor system and wherein said composition comprises at least $10^8$ attenuated or killed *methanobrevibacter Archaebacteria* cells per gram of said composition for use:

- in combination with a vaccination treatment of an animal subject;

- in a method for reducing susceptibility to inflammatory bowel disease (IBD) in humans;

- in a method for reducing immune pathology and disease severity caused by infections;

- in a method for promoting innate immune barrier functions and reducing susceptibility to parasitic infections in humans or animal subjects.

Other objects and advantages of the invention will become apparent to those skilled in the art from a review of the ensuing detailed description, which proceeds with reference to the following illustrative drawings, and the attendant claims.

## BRIEF DESCRIPTION OF THE DRAWINGS

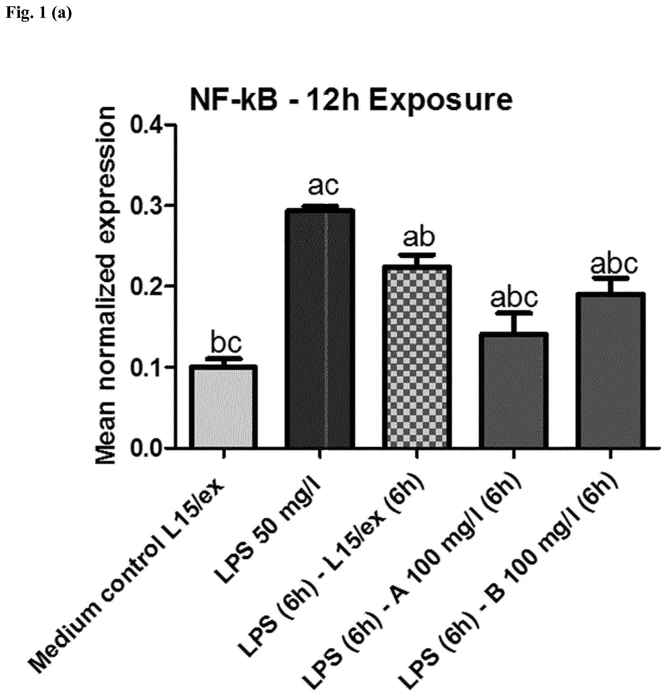

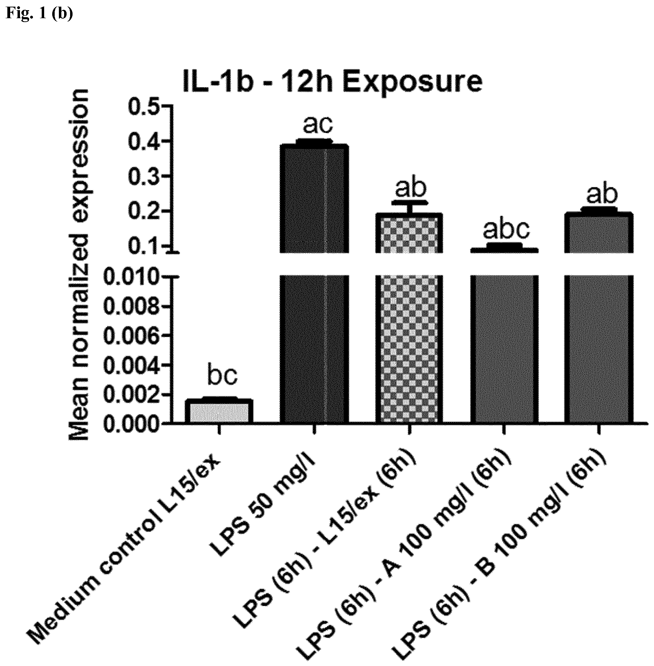

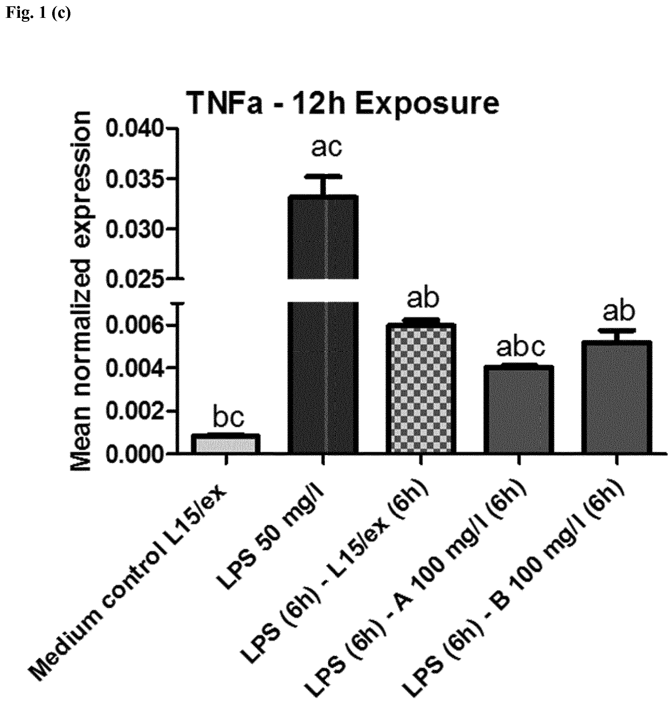

**Figure 1** illustrates that *M. Smithii* inhibits LPS mediated IL-1b, TNF-α and NF-kB in trout epithelial cells. Shown are the mean expression values and standard deviation measured in three biological triplicates of each treatment. Statistically significant differences from controls ((a) = comparison with negative control cell medium, (b) = comparison with positive control LPS 50 mg/l and (c) = comparison with LPS 50 mg/l 6h - L15/ex 6h) were determined with one-way analysis of variance (ANOVA), followed by Dunnett's test (P <0.05). Test items A) shows Cryo-Inactivated *M. smithii* archaea extract and B) High-Temperature-Inactivated M. smithii achaea extract.

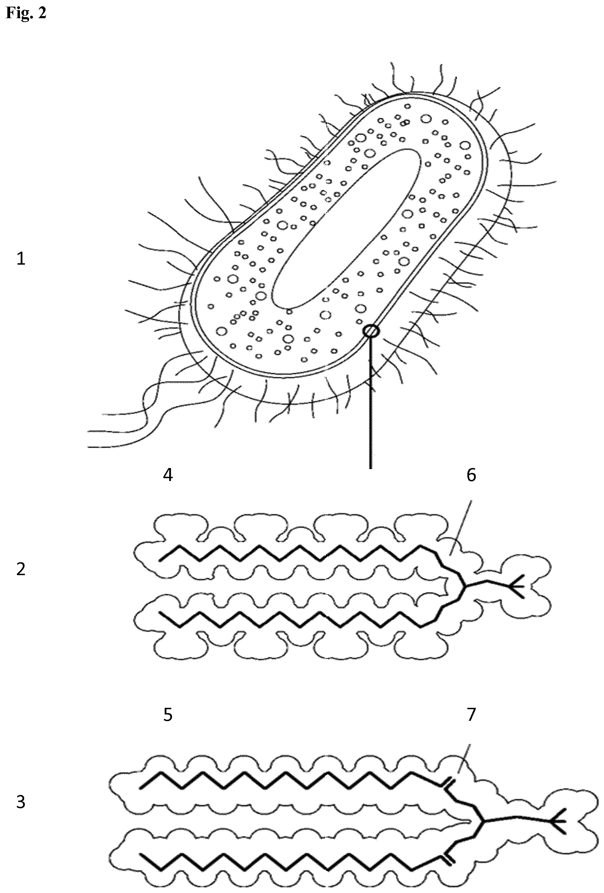

**Figure 2** illustrates an Archaea cell wall structure compared to bacteria cell wall structure. References in the figure are as follows: 1. Archaea (*M. smithii*); 2. Archaea lipid; 3. Bacteria lipid; 4. Branched isopropene chains; 5. Unbranched fatty acids; 6. Ether links and 7. Ester links.

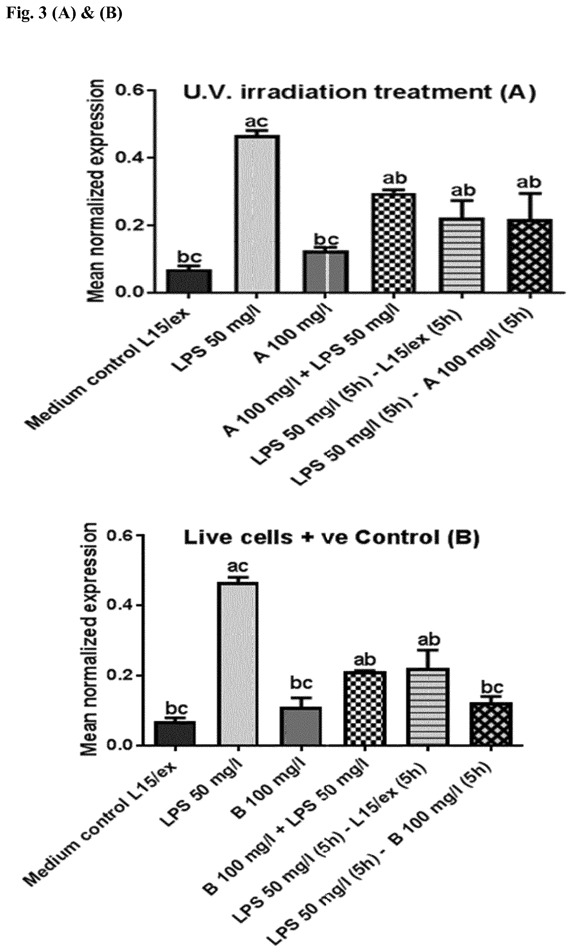

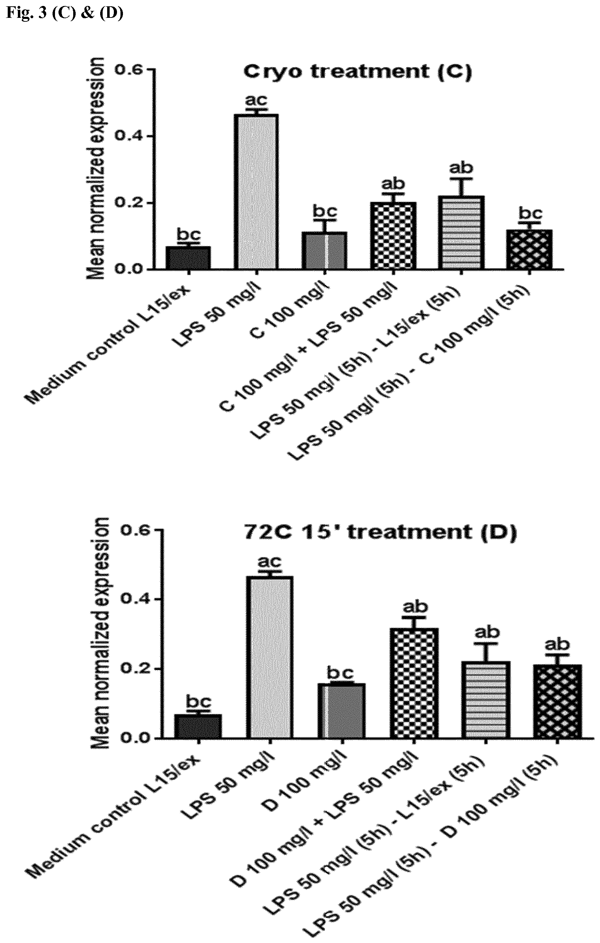

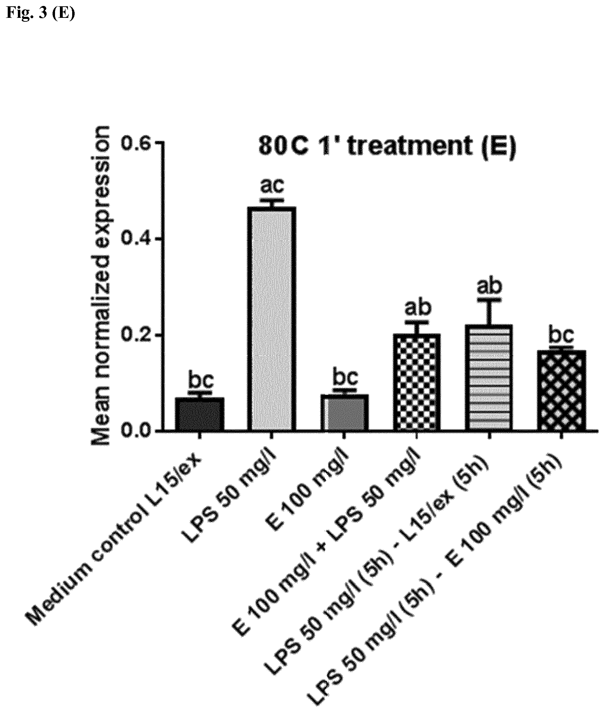

**Figure 3:** LPS induced NF-kB Expression is inhibited by live and inactivated Mbb archaea extract. (A) U.V. irradiation treatment, the resulting Mbb archaea extract does not inhibit the LPS induced NF-kB expression; (B) Live cells +ve control, this sample inhibits the LPS induced NF-kB expression; (C) Cryo treatment, the resulting Mbb archaea extract inhibits the LPS induced NF-kB expression; (D) 72°C 15' treatment, the resulting Mbb archaea extract does not inhibit the LPS induced NF-kB expression; (E) 80°C 1' treatment, the resulting Mbb archaea extract does inhibit the LPS induced NF-kB expression.

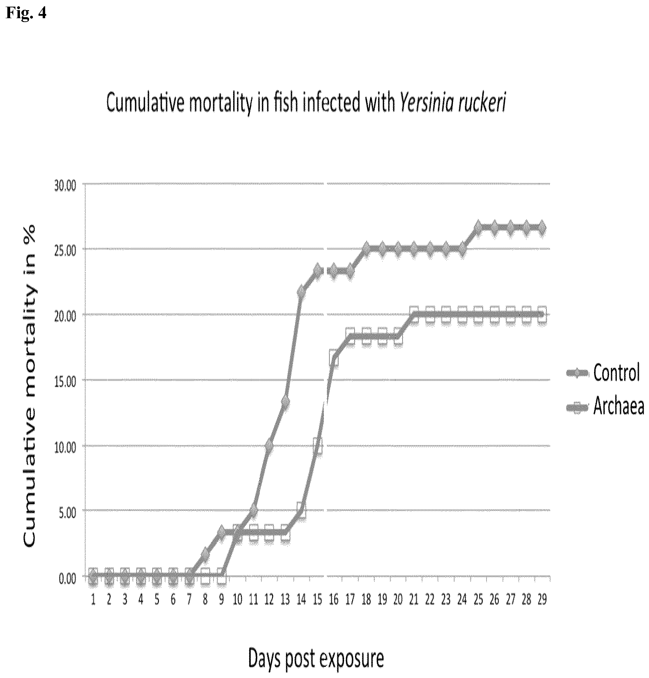

**Figure 4.** Cumulative mortality in fish infected with *Yersinia ruckeri*

## DETAILED DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control.

Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular forms "a", "an" and "the" include plural references unless the context clearly dictates otherwise.

In animal feeding, "fiber" or "fibre" is a term used to define a nutritional, not a chemical or plant anatomical concept. Fiber is the "indigestible and slowly digesting, or incompletely available, fractions of feeds that occupies space in the gastrointestinal tract" (Mertens, 1989), which defines fiber as insoluble components. Nutritionally, fiber has both physical and chemical attributes that are related to the mechanical processes of digestion (chewing and passage) and to enzymatic degradation associated with fermentation.

Ruminants such as cattle and sheep evolved as forage consumers. Plant cell walls, which we measure as fiber, cannot be digested by animals, but must be fermented by microorganisms. Fermentative digestion of fiber is slow and incomplete, and ruminants have developed many attributes that result in efficient digestion. They swallow large particles of forage and selectively retain them in the rumen to allow adequate time for fermentation. They regurgitate and rechew the large particles (rumination) to enhance digestion and allow passage through the digestive tract. During chewing, they produce salivary buffers that help maintain the pH in the rumen. Ideally, roughages could be an integral part of the diet of ruminants to take advantage of their unique digestive capability.

Fibrolytic archaea when delivered as a probiotic supplement, by forming symbiotic microbial communities, can help promote digestion of plant-based diets. Microbial communities colonize fiber in the intestine. These microbial communities that degrade plant fibers containing cellulose are referred to collectively as fibrolytic and cellulolytic microbes. Further, it is known that microbes that colonize fiber and their fermentation by-products are able to modulate immune cells since the majority of immune cells (white blood cells) in vertebrates are contained in the intestine.

The term "bioactive" refers to a compound that has an effect on a living organism, tissue /cell. In the field of nutrition bioactive compounds are distinguished from essential nutrients. While nutrients are essential to the sustainability of a body, the bioactive compounds or materials are not essential since the body can function properly without them, or because nutrients fulfil the same function. Bioactive compounds can have an influence on health.

"Prebiotics" are food ingredients that induce the growth or activity of beneficial microorganisms (e.g., bacteria and fungi). The most common example is in the gastrointestinal tract, where prebiotics can alter the composition of organisms in the gut microbiome. In diet, prebiotics are typically non-digestible fiber compounds that pass undigested through the upper part of the gastrointestinal tract and stimulate the growth or activity of advantageous bacteria that colonize the large bowel by acting as substrate for them.

As used herein, the term "probiotic" is a live microbial feed supplement which, when administered in adequate amounts, confer a health benefit on the host. The concept was introduced in the first part of the last century, by claiming that the dependence of the intestinal microbes on the food makes it possible to adopt measures to modify the flora in animal bodies and to replace the harmful microbes by useful microbes. Commonly claimed benefits of probiotics include the decrease of potentially pathogenic gastro-intestinal microorganisms, the reduction of gastro-intestinal discomfort, the strengthening of the immune system, the improvement of the skin's function, the improvement of bowel regularity, the strengthening of the resistance to cedar pollen allergens, the decrease in body pathogens, the reduction of flatulence and bloating, the protection of DNA, the protection of proteins and lipids from oxidative damage, and the maintaining of individual intestinal microbiota in subjects receiving antibiotic treatment.

A more detailed definition of probiotics relates to microorganisms that beneficially affect a host animal by modifying the host-associated or ambient microbial community, by insuring improved use of feed or by enhancing its nutrition, by enhancing the host response towards disease, or by improving quality of the ambient environment. This definition is especially appropriate when it comes to aquaculture. In fact, contrary to the terrestrial environment, where the gut represents a moist habitat in a water-limited world, in aquatic environments hosts and microorganisms share the ecosystem. Therefore, the environment for aquatic animals has much greater influence on microbiota than with terrestrials, and bacteria in aquatic medium heavily influence composition of host's gut microbiota. Aquatic animals are surrounded by an environment supporting their pathogens independently of the host animal, and opportunistic pathogens can therefore reach high densities around the fish, thus being commonly ingested with the feed or via drinking. Moreover, contrary to terrestrials which have inherent colonizing bacteria from the mother, aquatics largely spawned as axenic eggs. Ambient bacteria colonize eggs surface, and young larvae often have no developed gut (e.g., shrimp) and/or no microbial community in gut, gills or skin. As a consequence, since properties of bacteria in ambient water are very important, improvement of the ambient environment is crucial for the wellness of the bred animals.

Ruminants, such as cattle, rely upon a rich and diverse community of symbiotic ruminal microbes to digest their feed. These symbionts are capable of fermenting host-indigestible feed into nutrient sources usable by the host, such as volatile fatty acids. Rumen microbes can be assigned to different functional groups, such as cellulolytics, amylolytics, proteolytics, etc., which degrade the wide variety of feed components or further metabolize some of the products formed by other microbes.

"Fibrolytic microbiota" also referred herein as "Fiber-associated microbiota" especially "Rumen microbiota" consist in a symbiotic community of anaerobic microorganisms containing bacteria, protozoa, fungi and archaea that collectively degrade cellulose. The rumen microbiota is characterized by methanobrevibacter (Mbb) species that metabolize hydrogen by-products resulting from cellulose fermentation into methane. The terms rumen microbiota both refer to microbiota extracted from the rumen of a ruminant as well as artificial rumen microbiota prepared in laboratory by a skilled in the art.

More preferably, the fibrolytic microbiota culture or probiotic comprises at least one population selected from *Archaebacteria* species also referred herein as methanogenic *Archaebacteria* species.

The term "population" as used herein relates to a group of individual organisms of the same species defined by time and space. However, the term can be also intended as a community, i.e. a group of organisms inhabiting a particular ecological niche, which could include any number of species. In this context, the term "population" is also referred to as a "mixed population".

A list of "methanogenic *Archaebacteria* species" suitable in carrying out the invention comprises *Methanobacterium bryantii, Methanobacterium formicum, Methanobrevibacter arboriphilicus, Methanobrevibacter gottschalkii, Methanobrevibacter ruminantium, Methanobrevibacter smithii, Methanococcus chunghsingensis, Methanococcus burtonii, Methanococcus aeolicus, Methanococcus deltae, Methanococcus jannaschii, Methanococcus maripaludis, Methanococcus vannielii, Methanocorpusculum labreanum, Methanoculleus bourgensis, Methanoculleus marisnigri, Methanoflorens stordalenmirensis, Methanofollis liminatans, Methanogenium cariaci, Methanogenium frigidum, Methanogenium organophilum, Methanogenium wolfei, Methanomicrobium mobile, Methanopyrus kandleri, Methanoregula boonei, Methanosaeta concilii, Methanosaeta thermophila, Methanosarcina acetivorans, Methanosarcina barkeri, Methanosarcina mazei, Methanosphaera stadtmanae, Methanospirillium hungatei, Methanothermobacter defluvii, Methanothermobacter thermautotrophicus, Methanothermobacter thermoflexus, Methanothermobacter wolfei* and *Methanothrix sochngenii.* In one preferred embodiment, the *Archeabacteria* species is a *methanobrevibacter* (Mbb). Even more preferably, the *Methanobrevibacter* species used as active agent for the composition of the invention is the *Methanobrevibacter smithii* species.

The methanogenic *Archaebacteria* species produce methane as a metabolic by-product in anoxic conditions. Methanogens are a diverse group of strict anaerobes which are widely distributed in nature and can be found in a variety of permanently anoxic habitats like flooded soils, sediments, sewage-sludge digesters or the digestive tract of certain animals. All known methanogens are affiliated to the Archaea and extremely sensitive to oxygen. The hallmark feature of methanogens is the reduction of C-1 compounds (e. g., $CO_2$, methanol, formate, or N-methyl groups) to methane ($CH_4$). Methanogens play a vital ecological role in anaerobic environments of removing excess hydrogen and fermentation products that have been produced by other forms of anaerobic respiration. Methanogenic Archaea also play a pivotal role in ecosystems with organisms that derive energy from oxidation of methane, many of which are bacteria, as they are often a major source of methane in such environments and can play a role as primary producers. Methanogens also exert a critical role in the carbon cycle, breaking down organic carbon into methane, which is also a major greenhouse gas.

Methanogenesis also occurs in the guts of humans and other animals, especially ruminants. In the rumen, anaerobic organisms, including methanogens, digest cellulose into forms usable by the animal. Without these microorganisms, animals such as cattle would not be able to consume grass. The useful products of methanogenesis are absorbed by the gut, while methane is released by the animal.

The vertebrate gut is colonized by dense symbiotic communities of micro-organisms that support digestion and immunological fitness for their host. Throughout evolution, symbiotic micro-organisms are known to co-localize within their micro-environment; for instance, in ruminants (e.g. cows), micro-organisms promoting fiber digestion in the gut including yeast, cellulolytic bacteria and archaea, adhere and co-localize on individual grass fiber strands.

"Pathogen-associated molecular patterns", or PAMPs, are molecules associated with groups of pathogens that are recognized by cells of the innate immune system. These molecules can be referred to as small molecular motifs conserved within a class of microbes. They are recognized by toll-like receptors (TLRs) and other pattern recognition receptors (PRRs) in both plants and animals. A vast array of different types of molecules can serve as PAMPs, including glycans and glycoconjugates. PAMPs activate innate immune responses, protecting the host from infection, by identifying some conserved non-self-molecules.

Bacterial lipopolysaccharides (LPSs), endotoxins found on the cell membranes of gram-negative bacteria, are considered to be the prototypical class of PAMPs. LPSs are specifically recognized by TLR4, a recognition receptor of the innate immune system. Other PAMPs include bacterial flagellin (recognized by TLR5), lipoteichoic acid from gram-positive bacteria, peptidoglycan, and nucleic acid variants normally associated with viruses, such as double-stranded RNA (dsRNA), recognized by TLR3 or unmethylated CpG motifs, recognized by TLR9. Although the term "PAMP" is relatively new, the concept that molecules derived from microbes must be detected by receptors from multicellular organisms has been held for many decades, and references to an "endotoxin receptor" are found in much of the older literature.

The term "PAMP" has been criticized on the grounds that most microbes, not only pathogens, express the molecules detected; the term "microbe-associated molecular pattern" (MAMP), has therefore been proposed. A virulence signal capable of binding to a pathogen receptor, in combination with a MAMP, has been proposed as one way to constitute a (pathogen-specific) PAMP. MAMPs are essential structures for the microbes and are for that reason conserved. MAMPs include but not limited to lipopolysaccharide (LPS) cell membrane, Flagellin, Beta-Glycan, Peptidoglycan, Chitin etc... MAMPs are recognized by pattern recognition receptors (PRRs).

One object of the present invention is to provide a process for preparing a composition comprising attenuated or killed *methanobrevibacter Archaebacteria* cells for incorporation in human food/tablets and/or animal feed/tablets, the process comprising:

a) collecting a liquid suspension of *methanobrevibacter Archaebacteria* cells;
b) attenuating or killing said *methanobrevibacter Archaebacteria* cells in said liquid suspension;
c) freeze drying said liquid suspension to obtain an attenuated or killed *methanobrevibacter Archaebacteria* cell composition, wherein said attenuated or killed *methanobrevibacter Archaebacteria* cells have preserved at least 80% of the microbe-associated-molecular-patterns (MAMPS) signal potential through the host pattern recognition receptor (PRR) system.

As evidenced in example 4, Mbb inactivated according to the process of the invention proved capable of regulating the NF-kB reporter system indicating that the bioactive MAMPs were preserved post-inactivation treatment.

In Trine H. Mogensen "Pathogen Recognition and Inflammatory Signalling in Innate Immune Defenses" Clin Microbiol Rev. 2009 Apr; 22(2): 240-273: https://www.ncbi.nlm.nih.gov/pmc/articles/PMC2668232/ there is a general review on pattern recognition, including description of PRRs and NF-Kb inducible genes. This article also describes the benefit of TLR antagonists in therapy (effectively by inhibiting LPS induced inflammation what was done in example 4 with inactivated *methanobrevibacter* archaea extracts).

Serial dilutions of samples and comparative analysis to control MAMPs (e.g. LPS) represent methods to quantify MAMP signalling potential and the potency of the interaction with the host PRR system. In Corinna Bang et al. "The Intestinal Archaea Methanosphaera stadtmanae and Methanobrevibacter smithii Activate Human Dendritic Cells" PLOS ONE Vol. 9, no 6, E99411, 10 June 2014 (XP055590654) https://journals.plos.org/plosone/article?id=10.1371/journal.pone.0099411, 1 million archaea cells was capable of triggering signals from 200'000 Dendritic cells. These were not inactivated *methanobrevibacter* archaea cells but a serial dilution was performed. It is equivalent to 1 microgram of archaea for stimulation of 200'000 cells in vitro.

As it will be apparent to one skilled in the art, the amount of minimum value of MAMPs is relative. A purified MAMP such as LPS with a defined cognate receptor (TLR-4) can establish a dose threshold (mg/L) required for signal activation in a particular cell line. This can serve as a reference value and control for other MAMPs.

Since archaea extract contains a combination of MAMPs (proteins, cell wall fragments, glycosylated molecules) and is not a purified product such as commercially obtained LPS, the present invention refers to a minimum dose required to regulate NF-kB signalling in an *in vitro* model. In other cell lines, the minimum amount of MAMPs needed to trigger detectable signalling may be lower. Stimulation can also be conducted over a longer period of time and then the minimum dose of MAMP needed to trigger detectable signals will be reduced.

Thus the relative minimum amount of MAMP for a detectable signal is dependent on the cognate PRR of that MAMP (or combination of MAMPs) in a given cell line, the reporter cell type and duration of the stimulation.

In the context of the present invention, the minimum dose or amount is 10 mg/L or 1 microgram inactivated *methanobrevibacter* archaea MAMP product per 200'000 target cells for detection of biological signal in an *in vitro* cell assay.

Preferably, said inactivated of killed *methanobrevibacter Archaebacteria* cells have preserved 90%, ideally 95%, more preferably 99% and most preferably up to 100% of the microbe-associated-molecular-patterns (MAMPS) signal potential through the host pattern recognition receptor system.

Preferably, preservation of the microbe-associated-molecular-patterns (MAMPS) signal potential through the host pattern recognition receptor system is measured via NF-kB reporter pathway. NF-kB is a transcription factor that signals down stream of pattern-recognition receptors (PRRs). Measuring transcriptional regulation of NF-kB is a method to determine upstream pattern-recognition receptor interactions.

NF-kB is a global master regulator of inflammatory responses. Although NF-kB independent pathways exist, the NF-kB reporter model is an excellent method to determine pattern-recognition signalling potential.

Pattern-recognition receptors (PRRs) play a crucial role in the innate immune system of animals and humans. Pattern-recognition receptors are germline-encoded host sensors, which detect molecules typical of pathogens. They are proteins expressed by cells of the innate immune system, such as epithelial cells, to identify pathogen-associated molecular patterns (PAMPs) also referred to as microbe-associated molecular patterns (MAMPs). Lipopolysaccharide (LPS), is the major component of the outer membrane of Gram-negative bacteria. LPS is an important type of MAMP, which can induce strong inflammatory immune responses by activating host pattern-recognition receptors. In mammals, the pattern-recognition receptor TLR4 can recognize LPS and induce inflammatory responses. In fish, the pattern-recognition receptor NOD 1 can identify LPS and activate the NF-κB signaling pathway.

NF-κB (nuclear factor kappa-light-chain-enhancer of activated B cells) is a protein complex that regulates transcription of DNA and cytokine production. NF-κB plays a key role in regulating the immune response to infection. Incorrect regulation of NF-κB has been linked to cancer, inflammatory and autoimmune diseases.

Bacterial MAMPs through stimulation of cell-surface receptors lead to NF-κB activation and rapid changes in gene expression. For example, Toll-like receptors are pattern recognition molecules that lead to activation of NF-κB (TLR4 as the receptor for the LPS component of Gram-negative bacteria). The RNA expression level of NF-kB in *in vitro* cell cultures provides an effective reporter system for the activation of pattern-recognition receptors on the cells surface by different types of MAMPs.

MAMP signal potential through the host pattern recognition receptor (PRR) system refers to the capacity for MAMPs from a given product (e.g. cell wall from archaea extract) to interact with the PRR and thereby affect changes to the ensuing downstream signal cascade (e.g. NF-kB pathway, the regulation of NF-kB gene transcription). Only when MAMP signal potential through the host pattern recognition receptor system is preserved can signalling and regulation of downstream gene transcription (e.g. Nf-kB) be modulated.

In a preferred embodiment, the obtained attenuated or killed *methanobrevibacter Archaebacteria* cell composition of the invention is supplemented to a carrier originated from plants or a conventional carrier, preferably a biocompatible carrier.

Inactivated archaea can also be used in combination with a conventional carrier such as sucrose, sugar or starch based carrier as routinely used in freeze drying such as typical freeze-dry carrier materials.

By "carrier material" is meant the dosage form excipients, which provide the solid matrix support for the inactivated archaea. Inactivated *methanobrevibacter* archaea are incorporated within the matrix of the carrier material. The carrier material of the invention may include gelatin. Examples of suitable gelatin includes plain gelatin and gelatin that is partially hydrolyzed, for example by heating gelatin in water. For example, polysaccharide, plain gelatin, and hydrolyzed gelatin. Examples of other suitable carrier materials are those that are inert and pharmaceutically acceptable for use in food/feed/beverage or for preparing pharmaceutical dosage forms. Such carrier materials include polysaccharides such as dextran and polypeptides.

Preferably the plant carrier is a prebiotic. More preferably, the carrier originated from plants is selected among spirulina, clover, alfalfa, lucerne, oat, wheat, micro-algaes, macro-algaes, barley, corn, soy, rice (cereals, grasses, algaes etc, fruits, vegetables; materials containing cellulose, sugars, carbohydrates or chlorophyl) and/or extracts or mixtures thereof. Alternatively the carrier originated from plants is a fiber.

It is known, that the cell wall structure is a defining characteristic of archaea distinguishing them from bacteria. Archaea lipid cell walls are formed with branched isoprene chains joined by ether links. This is in contrast to bacteria lipid cell walls formed with unbranched fatty acids joined by ester links. This distinct cell wall biochemistry allows archaea to live in extreme environments. The archaea domain have evolved "extremophile" microorganisms. For example, the thermophile *Methanobrevibacter* species are known to thrive in temperatures exceeding 100°C. Such adaptations to extreme conditions are in part due to the cell membrane structure but also due to molecular chaperones including heat shock proteins. Certain structural and functional features of *M. smithii,* which have evolved to colonize the large intestine of herbivorous mammals, appear to be conserved as same as thermophiles (e.g. cell membrane structure).

During DNA extraction procedures, it has been observed that *Methanobrevacter smithii* species require harsher cell disruption procedures in order to isolate DNA. This observation is consistent with archaea having more robust cell wall structures.

Accordingly, it is essential that the process of attenuating or killing *methanobrevibacter Archaebacteria* cells of the composition according to the invention can preserve at least 80% of the microbe-associated-molecular-molecules (MAMPS).

Thus it is an object of the invention to provide a method of efficiently attenuating or killing *methanobrevibacter Archaebacteria* cells while preserving their immunological activity and function through the role of MAMPS.

In the context of the present invention, the terms "inactivation, inactivated or inactivating" refer to the process of killing a cell, whereby this cell after treatment is completely dead. Lyophilization is not a convenient technique that will allow 100% inactivation or killing of *methanobrevibacter Archaebacteria* cells and therefore this technique is not used in the inactivation step b) of the process of the invention.

According to another embodiment of the invention, "attenuated *methanobrevibacter Archaebacteria* cells" defined herein as cells that are no longer able to replicate (i.e. see example 4 § 3.2).

It is an important feature of the invention that the attenuating or killing of step b) is performed on a liquid suspension of *methanobrevibacter Archaebacteria* cells (wet step) otherwise; the inactivation (killing of the cells) will not be satisfactory. However, in cases when a combination of viable and non-viable cells is preferred, dry Mbb may be inactivated using industrial processes such as animal feed extrusion (sufficient high temperature exposure of the dry cells combined with steam and mechanical pressure). In the latter, the extruder operating conditions should be optimised to obtain the desired ratio of live to dead cells.

Any convenient liquid known to the skilled in the art for suspending *methanobrevibacter Archaebacteria* cells can be used. Examples of convenient liquids for suspending MBB are selected among: PBS, saline solution, water, sucrose solution, growth medium, rumen fluid, acetate, volatile fatty acid solutions etc..

In an embodiment of the invention the inactivation process of *methanobrevibacter Archaebacteria* cells is performed by pasteurization, cryo treatment, ultra-violet or infra-red irradiation or air drying techniques. Preferably, *methanobrevibacter Archaebacteria* cells are inactivated through cryogenisation or by pasteurization consisting in short heat pulse treatment.

"Cryogenisation" or "cryo treatment" or "cryo-inactivation" is a process of repeated freeze-thaw cycling so as to inactivate archaea cells. A method of cryo inactivating archaea cells is illustrated in example 2.

In the present invention, cryo-inactivation is usually performed with freeze cycles in range of -210C° to -20C° for at least 5 seconds, followed by thaw cycle in the range of 0C° to 37C° for at least 5 seconds. Freeze thaw cycles are performed at least twice.

In the case of liquid nitrogen cryopreservation for example, the temperature range would preferably be comprised between -196C° to -210C°.

"Air drying techniques" comprise a process of aeration so as to inactivate archaea cells. A method of inactivating archaea cells by air drying is illustrated in example 3.

In one embodiment, pasteurization preferably consisting in short heat pulse treatment is performed at a temperature from 55°C to 80 °C for at least 5 to 60 seconds or longer. Preferably pasteurization is performed for at least 20 seconds.

In another embodiment, ultra-violet irradiation is performed at a wave length from 10 to 400 nanometers.

Ultraviolet (UV) light is light with higher frequency (lower wavelength) than visible light. UV light has frequencies ranging from $8*10^{14}$ to $3*10^{16}$ Hz (800 THz to 30 PHz) and wavelengths from $10^{-8}$ m to $3.8*10^{-7}$ m (10 nm to 380 nm).

In yet another embodiment, infra-red irradiation is performed at a wave length from 750 nm to 1 millimeter.

The wavelength of infrared light is between 1 millimeter and 750 nanometers, while the wavelength of ultraviolet is between 400 nanometers and 10 nanometers. Infrared radiation extends from the nominal red edge of the visible spectrum at 700 nanometers (nm) to 1 millimeter (mm). This range of wavelengths corresponds to a frequency range of approximately 430 THz down to 300 GHz.

As would be also evident for a skilled person, a population of at least one *Archaebacteria* such as *methanobrevibacter* species can be obtained with any known method, such as purchase on the trade of isolated *Archaebacteria* strains (including lyophilized forms thereof), culture of *Archaebacteria* in suitable culture broths (such as for instance, the *Methanosphaera* Medium I or the *Methanobacterium* Medium from the Leibniz Institute DSMZ - German Collection of Microorganisms and Cell Cultures GmbH) with or without a pelleting step, and the like.

The rumen extract represents a perfect culture medium adjunct for *Archaebacteria* since it contains nutrients that nourish the microorganisms (especially methanogenic Archaea) under perfect culture conditions - anaerobic conditions in the cow rumen -. Through routine lab procedures, large amounts of rumen extract can be extracted from one cow per day; this can be possibly sterilized (through e.g. exposure to oxygen and/or extreme temperatures) and a rumen fluid obtained therefrom can be used as the basis to cultivate *Archaebacteria* under anaerobic, controlled lab conditions.

Preferably the inactivated Archaebacteria cells of the composition according to the invention are *methanobrevibacter smithii*.

It is another object of the invention to provide a composition comprising inactivated or killed *methanobrevibacter Archaebacteria* cells obtained according to the process of the invention, wherein said inactivated or killed *methanobrevibacter Archaebacteria* cells have preserved at least 80% of the microbe-associated-molecular-patterns (MAMPS) signal potential through the host pattern recognition receptor system and wherein said composition comprises at least $10^8$ inactivated or killed *methanobrevibacter Archaebacteria* cells per gram of said composition, for use in method for improving the resistance to intestinal pathogen infection and/or for reducing intestinal inflammation in a subject.

In a preferred embodiment, the composition of the invention comprises $10^9$, preferably $10^{10}$, more preferably

$10^{11}$ and even more preferably $10^{12}$ inactivated *methanobrevibacter* Archaebacterial cells per gram of said composition.

Preferably, said inactivated of killed *methanobrevibacter Archaebacteria* cells have preserved 90%, ideally 95%, more preferably 99% and most preferably up to 100% of the microbe-associated-molecular-patterns (MAMPS) signal potential through the host pattern recognition receptor system.

Preferably, preservation of the microbe-associated-molecular-patterns (MAMPS) signal potential through the host pattern recognition receptor system is measured via NF-kB reporter pathway.

In accordance with the invention, the subject is an animal or a human subject in needs thereof. Preferably, the animal is a bird, mammal or aquatic animal.

Preferably the subject is a farm animal. In other embodiment of the invention, the subject is a human.

In a preferred embodiment of the invention, the farm animals referred to in the present invention are birds, mammals or aquatic animals (such as shrimps, crustaceans or fishes). The invention is however not limited to this use. The composition of the invention may also be administrated to pets, captive animals or human beings.

Thus according to a specific embodiment, the composition of the invention is for use in human food such as for example a food supplement or tablet.

A "tablet" is a small disc or cylinder of a compressed solid substance, in the context of the present application typically a measured amount of inactivated *methanobrevibacter Archaebacteria* cells to be orally administered to a subject in need thereof.

In a preferred embodiment, the composition for use according to the invention is administrated at a dosage of at least $10^9$ inactivated or killed *methanobrevibacter Archaebacteria* cells per kilogram of said subject and per day. Preferably the composition is administrated at a dosage of at least $10^{10}$ inactivated or killed *methanobrevibacter Archaebacteria* cells, more preferably at a dosage of at least $2.10^{10}$ inactivated or killed *methanobrevibacter Archaebacteria* cells per kilogram of said subject and per day or even more.

In particular, the composition comprising inactivated *methanobrevibacter* archaea according to the invention can be used to reduce intestinal inflammation caused by food allergies/ insensitivities (gluten, lactose, etc...).

An advantage of inactivating *methanobrevibacter* Archaebacterial cells results in the possibility of incorporating more *methanobrevibacter* Archaebacteria cells i.e. in food/feed/tablet/beverage/suppository than it would be possible to do with living cells. This will consequently result in a better improvement of the resistance to intestinal pathogen infection and/or for reducing intestinal inflammation in a subject through the action and activity of microbe-associated-molecular-molecules (MAMPS) also present in inactivated *methanobrevibacter* Archaebacteria cells.

In an embodiment, the composition of the invention can be used in combination with a vaccination treatment of an animal subject.

Animals receive vaccines to prevent diseases. Vaccinating animals reduces animal suffering, reduces the transmission of microorganisms in the animal population, and is often more affordable than paying for the treatment of sick animals. Pets receive vaccines for infectious diseases such as rabies, parvovirus, distemper, and hepatitis.

Livestock animals like turkeys, chickens, cattle and pigs are vaccinated to protect against diseases like rotavirus, E. coli, pinkeye, and brucellosis. Vaccinations keep individual animals, flocks and herds, and people healthy. Animal vaccines are part of a category of animal medicines known as veterinary biologics, which work primarily by stimulating an animal's immune system to prevent or treat diseases. Vaccination treatment in combination with the composition of the invention reduces the side effects of vaccination and helps in restoring immunity.

According to another embodiment of the invention, the composition of the invention is used in the reduction of intestinal inflammation, which comprises reducing susceptibility to inflammatory bowel disease (IBD) in humans or animal subjects.

"Inflammatory bowel disease" (IBD) is an umbrella term used to describe disorders that involve chronic inflammation of the digestive tract. It is a complex disease and there can be different causes including sensitivity to diet. Inflammatory bowel disease (IBD) is a group of inflammatory conditions of the colon and small intestine. Crohn's disease and ulcerative colitis are the principal types of inflammatory bowel disease. It is important to note that not only does Crohn's disease affect the small intestine and large intestine, it can also affect the mouth, oesophagus, stomach and the anus whereas ulcerative colitis primarily affects the colon and the rectum.

In particular, the composition comprising inactivated *methanobrevibacter* archaea according to the invention can be used to reduce intestinal inflammation caused by food allergies/ insensitivities (gluten, lactose, etc...).

In addition, the composition comprising inactivated *methanobrevibacter* archaea according to the invention can be used as suppository (and for colon enema etc...) for modulation of the immune system without causing gas.

According to another embodiment, the composition of the invention is used in a method for reducing intestinal inflammation comprising reducing immune pathology and disease severity caused by infections in humans or animal subjects.

"Immune pathology" results from excessive or hypersensitive immune responses causing inflammation and damage to host tissue. Examples include cytokine release syndrome, eosinophilia, neutrophilia, types 1-4 hypersensitivity (allergic reactions etc...).

Immune pathology can be the result of autoimmunity (host immune responses targeting the host microbiota, host dietary antigens, host molecules). Inflammatory Bowel Disease (IBD) is an example of immune pathology.

Immune pathology can occur in higher and lower vertebrates though the mechanisms may differ since for example the mammalian immune system is more highly evolved than that of bony fish. IBD symptoms can occur in humans but also in lower vertebrates. For example, salmon when fed a plant rich diet, that they are not well adapted to, develop numerous IBD symptoms (inflammatory cytokines, intestinal swelling etc...). In 2015, an estimated 1.3% of US adults reported being diagnosed with IBD (https://www.cdc.gov/ibd/data-statistics.htm).

"Disease Severity caused by infections" refers to the level/extent of symptoms including: weight loss, epithelial lesions, swelling, pathogen load in tissues, fibrosis, faeces softness, behaviour (lethargy, posture, appetite), immune-markers in serum (inflammatory cytokines etc...). Disease severity caused by infection also refers to survival rates.

According to another embodiment, the composition of the invention is used in a method for promoting innate immune barrier functions and reducing susceptibility to parasitic infections in humans or animal subjects.

"The innate immune barrier" comprises multiple components/layers to circumvent invasion by pathogenic microorganisms and to contain pathogens and prevent their dissemination. The epithelial surfaces (e.g. lining the intestine) provide a mechanical barrier. The mucus layer (containing anti-microbial peptides, mucins, defensins and immunoglobulins) provides a biochemical barrier to inhibit pathogen invasion. Innate leukocytes (including macrophage, dendritic cells, granulocyte etc...) residing in the lamina propria are immune system sentinels that sense and secrete immune activating molecules (cytokines). Innate leukocytes also possess mechanisms to trap and neutralise invading microorganisms. Collectively, the multilayer innate immune barrier plays a critical role in protecting the host against infections.

Non-therapeutic indications to use the composition according to the invention are also encompassed, such as the improvement of immunological fitness of a subject.

"Immunity" is the quality or state of being immune; especially: a condition of being able to resist a particular disease especially through preventing development of a pathogenic microorganism or by counteracting the effects of its products.

The term "fitness" refers to general fitness (a state of health and well-being).

Physical fitness is the functioning of the heart, blood vessels, lungs, intestine and muscles to function at optimum efficiency, therefore, it is defined as the body's ability to func tion efficiently.

By improving "immunological fitness" it is meant a non-therapeutic method to:

- Resist infections,
- Tolerate/foster symbiotic microbes,
- Regulate the innate immune system,
- Regulate pattern-recognition signalling,
- Regulate appropriate cytokine networks,
- Regulate leukocyte distribution,
- Regulate mucosal immunoglobulin secretion,
- Regulate mucus quality,
- Regulate wound repair systems and maintaining integrity of host tissue ("house keeping" function).

In the context of the present invention, the improvement of the immune system function comprises the regulation of the intestinal homeostasis, the regulation of the inflammatory balance, the activation of immune genes and/or the immune recognition.

In accordance with the invention, the terms "intestinal homeostasis" refer to a state of mutualism when the intestinal immune system tolerates and fosters symbiotic microbiota. The intestinal immune system together with the symbiotic microbiota prevents the opportunistic over-growth of microbes also called 'dysbiosis'. Dysbiosis occurs when the microbiota composition is disturbed. Dysbiosis can activate the immune system to produce inflammatory factors (e.g. cytokines: IFN-g, TNF-a etc). This can disturb the normal (or regular) function of the intestine (thus the intestinal homeostasis is disturbed).

"Inflammatory balance" refers to regulation of cellular and molecular inflammatory networks. The primary function of inflammation is to eliminate pathogens. Inflammation while critical for resisting infection can cause damage to host tissues. Thus inflammation must be well regulated/balanced in order to ensure appropriate elimination of pathogens while minimizing immune pathology. Poorly regulated inflammation in some cases can be more harmful to the host than the damage caused by the pathogen per se.

The "activation of immune genes and/or the immune recognition" refers to the pattern-recognition system comprising receptors that sense the microenvironment for microbial patterns and regulate signaling cascades that control the immune system most commonly by producing cytokines (hormone type molecules of the immune system). The immune genes regulate white-blood-cell (WBC) proliferation, distribution and activation state. Pathogen-associated-molecular-patterns (PAMPs) are known to induce inflammation. The microbe-associated-molecular-patterns (MAMPs) of symbiotic microbiota are known to reduce inflammation and promote instead a state of tolerance in the intestine. Appropriate immune recognition and subsequent activation of appropriate immune genes is essential to proper immune system function.

Besides, it is submitted that none of the prior art such as US 2016/074440 A1 (Brugere et al.) demonstrate any applications of inactivated (killed) Mbb archaea (devoid of active TMA methyltransferase). Besides, Brugere et al. do not investigate the pattern-recognition receptor signaling induced by inactivated Mbb archaea.

The foregoing description will be more fully understood with reference to the following Examples. Such Examples, are, however, exemplary of methods of practising the present invention and are not intended to limit the scope of the invention.

**Examples**

**Example 1:**

**Inactivated *M. Smithii* archaea extract inhibits intestinal inflammation**

**1. Introduction**

Epithelial cells are important immune mediators and functional barriers in the vertebrate intestine.

LPS, derived from bacterial cell membrane, is a prototype inflammatory reagent.

IL-1b and TNF-a are both inflammatory cytokine markers induced by LPS stimulation of epithelial cells. NF-kB is a master regulator of inflammatory cytokines.

Aim:

In this study the immune modulating effect of inactivated *M. Smithii* was evaluated by exposure of *M. Smithii* to LPS activated trout epithelial cells and determining RNA expression levels of IL-1b, TNF-a and NF-kB.

**2. Material & Methods**

2.1 Cells

Cells of the RTgutGC (rainbow trout (Oncorhynchus mykiss) gut) cell line of passage 68 in 24- and 6-well plates. Per concentration, 3 replicate wells were dosed with 2 and 8 ml exposure solution per 24- and 6-well, respectively. Tests were performed at 19 $\pm$ 1 °C.

2.2 Marker Genes

Table 1: Selected immune response marker genes

| Gene | Organism |
|---|---|
| IL-1b (Interleukin-1 beta) | Rainbow trout (Oncorhynchus mykiss) |
| TNFa (Tumor necrosis factor alpha) | Rainbow trout (Oncorhynchus mykiss) |
| NF-kB (nuclear factor kappa-light-chain-enhancer of activatedB cells) | Rainbow trout (Oncorhynchus mykiss) |
| EF1a (Elogation factor 1 alpha)= Reference Gene | Rainbow trout (Oncorhynchus mykiss) |

2.3 RNA isolation / cDNA synthesis

Total RNA was extracted from the RTGutGC cells using Qiagen RNeasy Plus MiniKit.

Genomic:

DNA contaminations were removed using gDNA Eliminator spin column (Qiagen). RNA quantities and qualities were determined using a NanoDrop spectrophotometer (PEQLAB Biotechnologie GMBH). The cDNA was synthesized from total RNA using the High Capacity cDNA ReverseTranscription Kit (Applied Biosystems).

2.4 Quantification of marker gene mRNA expression

mRNA expression levels of all selected marker genes were analyzed using the GoTaq® qPCR Master Mix(Promega). All Samples were run in triplicates in white 384-well plates (Roche), using LightCycler480 (Roche). Transcript levels were measured in cDNAs of samples from independent RNA isolations. qPCR results were calculated relative to the housekeeping gene EF 1a, according to the normalization procedure of the Q-Gene Core Module (http://www.qgene.org/) which takes varying PCR amplification efficiencies into account.

2.5 Results gene expression analysis test item A & B:

Figures 1a, 1b, 1c: Gene expression results.
Shown are the mean expression values and standard deviation measured in three biological triplicates of each treatment.
Statistically significant differences from controls (a= comparison with negative control cell medium, b = comparison with positive control LPS 50 mg/l and c = comparison with LPS 50 mg/l 6h - L15/ex 6h) were determined with one-way analysis of variance (ANOVA), followed by Dunnett's test (P <0.05).

**Results:**

Exposure of trout epithelial cells to LPS 50mg/L, a standard inflammatory reagent, for 6 hours resulted in strong increased gene expression of the inflammatory marker genes NF-kB, IL-1b and TNF$\alpha$ compared to L15/ex medium control. Removal of LPS and replacement with 100mg/L of test item A (Cryo-Inactivated M. smithii archaea extract) resulted in a significant reduction in the expression level of all inflammatory markers compared to replacement with L15/ex medium control. In contrast, test item B (High-Temperature-Inactivated M. smithii archaea extract) resulted only in significant reduction of NF-kB expression but not IL-1b nor TNF-$\alpha$. Inactivated M. Smithii archaea extract inhibited LPS mediated inflammation. The cryo-inactivated M. smithii archaea extract had broader and more potent anti-inflammatory effects than the high-temperature inactivated M. smithii archaea extract.

**Figure 1a, 1b, 1c: M. Smithii inhibits LPS mediated IL-1b, TNF-$\alpha$ and NF-kB in trout epithelial cells**

Test items:

Cryo-Inactivated *M. smithii* archaea extract
High-Temperature-Inactivated *M. smithii* achaea extract

Gene expression results:

Shown are the mean expression values and standard deviation measured in three biological triplicates of each treatment. Statistically significant differences from controls (a= comparison with negative control cell medium, b = comparison with positive control LPS 50 mg/l and c = comparison with LPS 50 mg/l 6h - L15/ex 6h) were determined with one-way analysis of variance (ANOVA), followed by Dunnett's test (P <0.05).

**Discussions:**

LPS mediated inflammation is widely known to be mediated via Toll-like-receptor 4 (TLR4) a major pattern recognition receptor as well as NOD-1. The study shows that inactivated archaea extract inhibits LPS mediated inflammation. Moreover, cryo-inactivated archaea extract is more potently anti-inflammatory than high-temperature inactivated archaea extract. These data demonstrate that inactivated archaea can retain immunological active MAMPs after inactivation if appropriately processed. It seems expected that archaea MAMPs interfere with LPS -TLR4 mediated inflammation, possibly through competitive cross-linking with TLR4.
Appropriately inactivated archaea extract that retain MAMPs show excellent potential to reduce immune pathology as well as show excellent potential to promote immune homeostasis in the intestine as well as systemically to reduce the risk for dysbiosis, infection and disease.

**Conclusions:**

Inactivated *M. smithii* archaea of the *Methanobrevibacericeae* family demonstrate anti-inflammatory properties in intestinal epithelial cells. Cryo-inactivation of *M. smithii* archaea extract provides even better preservation of immune functional MAMPs than high-temperature-inactivated *M. smithii* archaea extract.

**Example 2:** Cryogenisation

10g of live centrifuged *M. smithii* archaea biomass are transferred to a 15 mL falcon tube. The live centrifuged archaea is snap frozen by immersion of the falcon tube in liquid nitrogen for 5 minutes. Next, the falcon tube containing frozen archaea is thawed for 10 minutes in a water bath maintained at 13C°. This freeze-thaw cycle is performed 3-10 more times.

The inactivated archaea extract can be immediately used or alternatively preserved by, for example, routine freeze-dry formulation and treatment.

The resulting powder contains pure inactivated *M. smithii* archaea extract. The non-viability of *M. smithii* is verifiable using routine *M. smithii* anaerobic culturing methods as well as the Live/Dead BacLight Bacterial Viability Kit from Thermofisher.

**Example 3:** Air drying

10g of live centrifuged *M. smithii* archaea biomass are smeared thinly over nylon mesh strainer with 10 cm diameter (40 - 500 micron, preferably 70 micron pores). Warm air 37C° is then blown over (e.g. using an air dryer device, industrial oven etc...) the surface of the biomass for drying. Drying for 2 hours, mixing the biomass gently with a flat surface against the nylon mesh at 20 minute intervals provides homogeneity, to ensure sustained exposure of all cells in the biomass to air. The prolonged exposure of *M. smithii* to air for 1-5 hours after harvest inactivates the cells. After the air dry process, the inactivated archaea extract can be immediately used or alternatively preserved by, for example, routine freeze-dry formulation and treatment.

The resulting powder contains pure inactivated *M. smithii* archaea extract. The non-viability of *M. smithii* is verifiable using routine *M. smithii* anaerobic culturing methods as well as the Live/Dead BacLight Bacterial Viability Kit from Thermofisher.

**Example 4:**

Methods for inactivation of Methanobrevibacter (Mbb) archaea capable of preserving the microbe-associated-molecular-patterns (MAMPS) signal potential through the host pattern recognition receptor system

**1. Introduction**

1.1. The tolerance of Methanobrevibacter (Mbb) to thermo

*Methanobrevibacter* (Mbb), a genus in the domain of archaea, have emerging commercial applications in the life-sciences (e.g. as probiotics). Since Mbb are strict anaerobes the tolerance of most species to air is assumed to be low. Moreover, Mbb indigenous to the intestine of herbivores are generally adapted to physiological temperatures in contrast to thermophile Mbb. It remains unclear to what extent intestinal Mbb are tolerant to air and extreme temperature exposure.

The cell wall structure is a defining characteristic of archaea distinguishing them from bacteria (Figure 2). Archaea lipid cell walls are formed with branched isoprene chains joined by ether links. This is in contrast to bacteria lipid cell walls formed with unbranched fatty acids joined by ester links. This distinct cell wall biochemistry allows archaea to live in extreme environments. The archaea domain have evolved 'extremophile' microorganisms. For example, the thermophile *Methanobrevibacter* species are known to thrive in temperatures exceeding 100°C. Such adaptations to extreme conditions are in part due to the cell membrane structure but also due to molecular chaperones including heat shock proteins. Certain structural and functional features of *M. smithii,* which have evolved to colonize the large intestine of herbivorous mammals, appear to be conserved as same as thermophiles (e.g. cell membrane structure).

During DNA extraction procedures, it has been observed that *M. smithii* species require harsher cell disruption procedures in order to isolate DNA. This observation is consistent with archaea having more robust cell wall structures.

Due to conservation of extremophile features (e.g. cell wall structure) the tolerance of Mbb to thermo treatments, air and irradiation remains unclear. It is known that archaea are resistant to antibiotic treatment due to the distinct cell wall structure. Viability after the treatments can be evaluated using live-dead staining and culturing techniques. Moreover, the effect of such treatments on microbe-associated molecular patterns (MAMPs), which interact with the intestinal immune system of recipient animals, remains unclear.

1.2. Archaea MAMP-PRR interactions post-inactivation treatment not known

Pattern-recognition receptors (PRRs) play a crucial role in the innate immune system of animals. Pattern-recognition receptors are germline-encoded host sensors, which detect molecules typical of pathogens. They are proteins expressed by cells of the innate immune system, such as epithelial cells, to identify pathogen-associated molecular patterns (PAMPs) also referred to as microbe-associated molecular patterns (MAMPs).

Lipopolysaccharide (LPS) is the major component of the outer membrane of Gram-negative bacteria. LPS is an important type of MAMP, which can induce strong inflammatory immune responses by activating host pattern-recognition receptors. In mammals, the pattern-recognition receptor TLR4 can recognize LPS and induce inflammatory responses. In fish, the pattern-recognition receptor NOD1 can identify LPS and activate the NF-κB signaling pathway.

NF-κB (nuclear factor kappa-light-chain-enhancer of activated B cells) is a protein complex that regulates transcription of DNA and cytokine production. NF-κB plays a key role in regulating the immune response to infection. Incorrect regulation of NF-κB has been linked to cancer, inflammatory and autoimmune diseases.

1.3. MAMP-PRR signalling: the NF-kB reporter model

Bacterial MAMPs through stimulation of cell-surface receptors lead to NF-κB activation and rapid changes in gene expression. For example, Toll-like receptors are pattern recognition molecules that lead to activation of NF-κB (TLR4 as the receptor for the LPS component of Gram-negative bacteria).

The RNA expression level of NF-kB in in vitro cell cultures provides a clear reporter system for the activation of pattern-recognition receptors on the cells surface by different types of MAMPs.

In this study, Applicants demonstrate thresholds required to inactivate a dominant intestinal Mbb prototype, *M. smithii,* and evaluate the capacity of MAMPs derived from the inactivated archaea extract to inhibit LPS induced NF-kB activation in rainbow trout epithelial cells.

**2. Materials & Methods**

2.1. Mbb treatments

A strain of *M. smithii* (Mbb) obtained from DSMZ was cultured in anaerobic bioreactors.

*At stationary growth phase, cells were harvested and rinsed twice with PBS to remove all traces of medium.

The cells were then exposed to various treatments as described below and their viability assessed using live-dead cell staining and culturing methods.

2.1.1. Live cells, positive control

In a 100 ml sterile serum bottle prepare a suspension of 0.01 g wet Mbb biomass in 20 ml growth medium -> evaluate viability

Suspend remaining cells in 5 mL 10% sucrose solution in PBS
Snap freeze
Store on dry ice and then freeze dry.

2.1.2. U.V. inactivation treatment

Suspend 5 g of wet Mbb biomass in 7 L PBS for U.V. irradiation.

After irradiation (irradiation parameters: 2KJ/L radiation dose, treatment time 40 sec), centrifuge, pellet.

In a 100 ml sterile serum bottle prepare a suspension of 0.01 g wet Mbb biomass (from the new UV treated pellet) in 20 ml growth medium -> evaluate viability Suspend remaining cells in 5 mL 10% sucrose solution in PBS

Store on dry ice and then freeze dry.

2.1.3. 72°C for 15' - sustained heat treatment

Place 5g wet Mbb biomass at 72°C for 15 minutes (put tube in water bath)
In a 100 ml sterile serum bottle prepare a suspension of 0.01 g wet Mbb biomass in 20 ml growth medium -> evaluate viability
Suspend remaining cells in 5 mL 10% sucrose solution in PBS
Snap freeze
Store on dry ice and then freeze dry.

2.1.4. 80°C for 1' - pulse heat treatment

Place 5g wet Mbb biomass at 80°C for 1 minute (put tube in water bath)
In a 100 ml sterile serum bottle prepare a suspension of 0.01 g wet Mbb biomass in 20 ml growth medium -> evaluate viability
Suspend remaining cells in 5 mL 10% sucrose solution in PBS
Snap freeze
Store on dry ice and then freeze dry.

2.1.5. Cryo-inactivation treatment

Place 5g wet Mbb biomass in liquid nitrogen (LN) for 10 minutes
Transfer cells to RT for 10 minutes (until thawed)
Repeat freeze-thaw (LN <-> RT) 10 more times
In a 100 ml sterile serum bottle prepare a suspension of 0.01 g wet Mbb biomass in 20 ml growth medium -> evaluate viability
Suspend remaining treated cells in 5 mL 10% sucrose solution in PBS Store on dry ice and then freeze dry.

2.1.6. Attenuation by exposure of wet cells to air

Sparging air in bioreactor for 10'.

2.2. Viability testing

Viability and inactivation of archaea following various treatments was evaluated using live-dead staining and culturing methods as described below.

2.2.1. Live-dead staining

Cell viability was evaluated using Live/dead Baclight® kit L7012 (ThermoFisher Scientific, Switzerland)

2.2.1.1. Flow cytometry

*M. smithii* cells were stained with the Live/dead Baclight® kit L7012 (ThermoFisher Scientific, Switzerland) containing Syto 9 and propidium iodide (PI) according to the manufacturer instructions. Briefly, 250 µL of *M. smithii* suspensions were centrifuged at 2000 x g or 3000 x g for 5 min, respectively, the supernatants discarded and the pellets resuspended with the same initial volume of 0.85% NaCl. The suspensions were adjusted to approximately $1 \times 10^8$ microorganisms/mL (≈0.03 OD670), and further diluted 1:100 in 0.85% NaCl to reach a final density of ≈1 x 106 microorganisms/mL. Separately, 1 part of Syto 9 was mixed with 1 part of PI, and 0.75 µL of the dyes mixture were added to 250 µl of each microbial suspension and the final mixture incubated for 20 min at room temperature in the dark. Duplicates were prepared for the controls and triplicates for the samples.
When performing an absolute count, BD TrucountTM Tubes (BD Biosciences, Switzerland) were used. An aliquot of each time point replicate was centrifuged and handled as aforementioned. The final 250 µL of the suspension at ≈1 x 106 microorganisms/mL were prepared in the corresponding TrucountTM Tube (1 tube per each sample) following the instructions of the manufacturer, the 0.75 µL of the Syto 9 + PI mixture were added, and the suspension incubated for 20 min at room temperature in the dark.
Flow cytometry acquisition was performed at the Flow Cytometry Core Platform of the Faculty of Medicine (University of Geneva, Switzerland). Acquisition was carried out with a BD Accuri™ C6 flow cytometer (BD Biosciences, Switzerland), equipped with a 488 nm-excitation laser in channels FL-1 to FL-3 and a 635 nm-excitation laser in channel FL-4. Forward (FSC) and side scatter (SSC) were collected from 488 nm excitation through a 488/10 nm band pass filter. Syto 9 was excited with the 488 nm-laser and its emission was collected on the FL-1 channel with a band pass filter at 533/30 nm. PI was excited with the 488 nm-laser and its emission was collected through the 670 nm-long pass filter on FL-3 channel. Beads of TrucountTM tubes for absolute counting were excited with the 635 nm laser and their red fluorescence collected through a 675/25 nm-band pass filter in the FL-4 channel. No voltage compensation was set. Cells were gated from SSC vs FL-1 plots because they were better discriminated from the noise by their fluorescence than by their size (FSC channel). For each sample, 10'000 events in the "cells" gate were recorded. Data was acquired with the BD Accuri C6 software.

2.2.1.2. Flow cytometry data analysis

Flow cytometry data was analyzed with FlowJo® software (Version 10; ©FlowJo LLC, USA) and BD Accuri C6 software was also used when performing absolute counting. No elimination of doublets was necessary for acquisitions with this equipment. Live and dead controls served to place the gates for "live" and "dead" populations, respectively. Another gate named "injured" was created for the events appearing between the "live" and "dead" gates. The percentage of cells in each gate or population was given by the analysis software.

The absolute concentration of live cells in each sample was determined from the number of events counted in the live gate according to Equation 3:

$$\frac{n°\text{ "live" events}}{n°\text{ beads events} \times \dfrac{V sample}{n°total\ beads}} \times 1000 \times F$$

where F is the dilution factor from the concentration in the flask to the analyzed fraction by flow cytometry.

2.2.1.3. Fluorescence microscopy

Centrifuged *M. smithii* suspensions (in the same conditions as for flow cytometry) and resuspended in 0.85% NaCl were further diluted 1: 10 with 0.85% NaCl (50 μL prepared). 0.15 μL of the Syto 9/PI mixture were added to the cell suspension and incubated for 20 min at room temperature in the dark. Subsequently, 4 μL of each sample were deposited on a slide (76 x 26 mm) and covered with a coverslip (22 x 22 mm), which was sealed with nail polish. Image acquisition and image analysis were performed on the Bioimaging Core Facility of the Faculty of Medicine (University of Geneva, Switzerland). An AxioCam 506 mono detector (Zeiss, Feldbach, Switzerland) mounted on a Zeiss Axio Imager Z1 (Zeiss) was used. It was mounted with a 63x/1.4 NA oil Plan-Apochromat objective having a resolution of 0.072 x 0.072 μm. Syto 9 was excited and detected through an FITC (green) filter set composed of a BP 450-490 for excitation, an FT 510 as beam splitter and a BP 515-565 for emission. PI was excited and detected through a Cy3 (red) filter set composed of a BP 546/12 for excitation, an FT 580 as a beam splitter and a LP 590 for emission. The exposure time was set at 120 ms for the green channel and 150 ms for the red channel. The images were recorded and treated with the software Zen (version 2.3, Zeiss) on a PC operating with Windows 10 OS. Images shown were treated using an interpolation filter.

2.2.1.4. Statistical analyses

The extent of growth in *M. smithii* after exposure at a given temperature was assessed by determining the area under the curve (AUC) of the OD600 vs time graph between day 0 after treatment and day 6. The significant differences between the AUC mean values were assessed by a one-way ANOVA and a Tukey HSD test for multiple comparison of the means. Differences were considered significant when the P value was < 0.05.

Determining viable cell culture by O.D. readings

Take 2 mL sample of cell suspension and place it into a cuvette. (The blank is measured against water). Measure the optical density (OD) at a wavelength of 600nm. The linear absorbance range is between 0.1 and 0.3 - if the value measured is higher than the range, the sample must be dilute in DI water.

The optical density is directly proportional to the biomass in the cell suspension in a given range.

2.3. NF-kB reporter system

NF-kB is a transcription factor that signals down stream of pattern-recognition receptors (PRRs). Measuring transcriptional regulation of NF-kB is a method to determine upstream pattern-recognition receptor interactions.

2.3.1. Cells

Cells of the RTgutGC (rainbow trout (Oncorhynchus mykiss) gut) cell line of passage 68 in 24- and 6-well plates. Per concentration, 3 replicate wells were dosed with 2 and 8 ml exposure solution per 24- and 6-well, respectively. Tests were performed at 19 ± 1 °C.

Table 2

|  | **RTgutGC cell line assay** |
|---|---|
| Assignment and objective: | Performance of the RTgutGC cell line assay, including gene expression analysis of rainbow trout immune activation markers |
| Application of the test items: | A stock solution of 1000 mg/l in exposure medium (L-15/ex) was prepared for each of the test items. |
| Exposure conditions for each test item: | a) Test item 100 mg/l for 10 h<br>b) Test item 100 mg/l + LPS( bacterial lipopolysaccharide) 50 mg/l for 10 h<br>c) LPS 50 mg/l for 5h → removal of LPS → Test item 100 mg/l for 5 h |
| Control exposure conditions | a) Medium control L-15/ex for 10 h<br>b) LPS 50 mg/l for 10 h<br>c) LPS 50 mg/l for 5h → removal of LPS → L-15/ex for 5 h<br>d) LPS 50 mg/l for 5h → removal of LPS → Pen-Strep for 5 h |
| Test System: | Cells of the RTgutGC (rainbow trout (*Oncorhynchus mykiss*) gut) cell line of passage 68 in 6-well plates. Per exposure condition, 3 replicate wells were dosed with 8 ml exposure solution per well, respectively. |
| Temperature: | 19 ± 1°C |
| Exposure duration: | 10 hours |

2.3.2. Marker Genes

Table 3: Selected immune response marker gene and reference gene

| Gene | Organism |
|---|---|
| NF-kB (nuclear factor kappa-light-chain-enhancer of activated B cells) | Rainbow trout (Oncorhynchus mykiss) |
| EF1a (Elogation factor 1 alpha)= Reference Gene | Rainbow trout (Oncorhynchus mykiss) |

2.3.3. RNA isolation / cDNA synthesis

Total RNA was extracted from the RTGutRC cells using Qiagen RNeasy Plus MiniKit. Genomic DNA contaminations were removed using gDNA Eliminator spin column (Qiagen). RNA quantities and qualities were determined using a NanoDrop spectrophotometer (PEQLAB Biotechnologie GMBH) (see appendix). The cDNA was synthesized from total RNA using the High Capacity cDNA ReverseTranscription Kit (Applied Biosystems).

2.3.4. Quantification of marker gene mRNA expression

mRNA expression levels of all selected marker genes were analyzed using the GoTaq® qPCR Master Mix(Promega). All Samples were run in triplicates in white 384-well plates (Roche), using LightCycler480 (Roche). Transcript levels were measured in cDNAs of samples from independent RNA isolations. qPCR results were calculated relative to the housekeeping gene EF1a, according to the normalization procedure of the Q-Gene. Core Module (http://

www.qgene.org/) which takes varying PCR amplification efficiencies into account.

2.3.5. Results gene expression analysis test items

Shown are the mean expression values and standard deviation measured in three biological triplicates of each treatment. Statistically significant differences from controls (a= comparison with negative control cell medium, b = comparison with positive control LPS 50 mg/l and c = comparison with LPS 50 mg/l 6h - L15/ex 6h) were determined with one-way analysis of variance (ANOVA), followed by Dunnett's test (P<0.05).

## 3. Results

3.1. Inactivation thresholds were determined and pattern-recognition signalling potential tested

Lyophilized Mbb was resistant to extreme heat treatment. The inactivation required Mbb cells first to be suspended in a wet culture prior to treatment. A summary of the Mbb inactivation thresholds is displayed in table-2. Also displayed in table-2, the effect of the treatment type on Nk-kB signalling potential.

Table 4: Summary

| Mbb inactivation threshold and corresponding MAMP signal potential | | | | |
|---|---|---|---|---|
| | | Mbb | | |
| | Treatment | Live Stain | Viable culture | NF-kB signal |
| Wet cells | U.V. 2KJ/L | - | - | - |
| | +ve Control | + | + | + |
| | Cryo | - | - | + |
| | 72°C, 15' | - | - | - |
| | 80°C, 1' | - | - | + |
| Lyophilized | 80°C, 30' | + | + | + |

Inactivation of Mbb by either cryo treatment or short heat pulse (80°C for 1 minutes) were both methods that preserved interaction with the NF-kB reporter signalling pathway indicating that the MAMP signal potential was preserved when using these treatment methods.
In contrast, inactivation of Mbb using either U.V. irradiation or sustained heat (72°C for 15 minutes) did not preserve interaction with the NF-kB pattern-recognition.

3.2. Attenuation by exposure of cells in culture to air

A 10 minute exposure of wet Mbb cells to air (oxygen) by vigorous sparging of the growth media produces an attenuated state. Cells display live features (F420 enzyme and live cell staining features) but cannot replicate due to accumulation of air in the cell. In contrast, lyophilized dry cells are tolerant to air as they do not absorb the air in the cell.

3.3 Mbb inactivation treatments that preserve pattern-recognition signalling via NF-kB reporter pathway.

Statistical significance of the results illustrated in Figure 3 (A)-(E):

A) Significant differences to Medium Control L-15/ex:

LPS, Test Item 100 mg/l + LPS,
LPS (5h) - L-15/ex (5h), LPS (5h)- P/S 0.1
mg/ml(5h), LPS (5h)- Test Item 100 mg/l (5h)

B) Significant differences to LPS Control:

L-15/ex, P/S 0.1 mg/ml, Test Item 100 mg/l, Test Item
100 mg/l + LPS, LPS (5h) - L-15/ex (5h), LPS (5h)- P/S

0.1 mg/ml(5h), LPS (5h)- Test Item 100 mg/l (5h)

C) Significant differences to LPS 50 mg/l 5h - L-15/ex 5h :
L-15/ex, LPS, P/S 0.1 mg/ml, Test Item 100 mg/l

**Conclusion**

Inactivation thresholds of Mbb were characterized. Short heat pulse or cryo treatment were both methods that preserved functional fragments involved in inhibiting LPS induced inflammation.

Mbb inactivated according to the method of the invention proved capable of regulating the NF-kB reporter system indicating that the bioactive MAMPs were preserved post-inactivation treatment.

**Example 5: Experiment to test the effect of inactivated archaea extract on the course of red mouth disease in rainbow trout (Oncorhynchus mykiss)**

**Background**

Aquaculture is one of the fastest growing industries worldwide and produces an important part of the world's protein resources. However, different factors influence the productivity of farming efforts among them diseases. In intensive fish farming conditions bacterial diseases can have devastating effects. To prevent infections or reduce their effect different approaches have to be considered. In view of increasing problems with bacteria resistant to antibiotics alternatives are needed and highly welcome. One potential approach is to feed farmed animals with diets containing additives which have a beneficial effect on the intestinal immune system are able to reduce the harming effect of bacterial infections.

**Aim**

The present example aimed at testing the effect of inactivated *Methanobrevibacter* (Mbb) archaea extract on the course of Enteric Red Mouth Disease (ERM) in rainbow trout. Fish were challenged with Yersina ruckeri, the etiologic agent of ERM and the course of the disease in the group receiving inactivated archaea extract was compared to that of a group receiving a control diet.

**Material and Methods**

**Fish:**

Young rainbow trout of 29 + 1 gram body weight were obtained and fed control and experimental diet (namely the composition of the invention) for 1 month.

**Tanks:**

For the acclimatization and the following period until start of the challenge experiment 130 litre glass aquaria equipped with a tap water flow-through system and constant aeration were used. Water temperature during the whole experiment was maintained at 15.5 ± 1.0°C.

For the challenge experiment fish were transferred to 38 litre glass aquaria equipped with a flow-through system with tap water and constant aeration. Water temperature was maintained at 15.5 ± 1.0°C.

Fish were fed daily at a rate of 1% of the body weight. The diet rate was regularly adjusted to the weight gain of fish. In the challenge experiment the diet rate was daily adjusted to the number of surviving fish in every tank

**Test diets:**

All diets were fed from the arrival of fish until the end of the experiment.

Table 5: Diets tested in the experiment

| Diet de-signation | Specific content |
|---|---|
| Control | Control diet |

(continued)

| Diet de-signation | Specific content |
|---|---|
| Archaea extract treatment | Diet supplemented with inactivated Mbb archaea extract (Dosed at $10^9$ inactivated Mbb cells per Kg fish per day) |

Mbb was prepared by the Applicant. Pure culture of M. Smithii was harvested and enumerated using cell counting chambers. Wet cells were inactivated using thermal treatment (verified by BAC Light live dead staining method and O.D. culture method) and then freeze-dried prior to inclusion in fish feed pellets.

**Handling of fish**

Upon arrival, fish were weighed per group and tank. Subsequently, fish were weighed when transferred to the experimental tanks for the challenge experiment. Based on the obtained data the mean dose of diet per tank was calculated and adjusted accordingly.

**Bacteria**

A laboratory isolate and an isolate of Yersinia ruckeri (designation 17/Ref0264) obtained from the Institute of Veterinary Bacteriology (IVB) were used. The latter isolate had originally been isolated from rainbow trout from a commercial fish farm.

Bacteria were grown in LB-broth at 25°C and harvested after 18 h. Density of bacteria was then assessed photospectrometrically and the concentration adjusted to the needed values with PBS.

**Determination of challenge dose**

In order to determine a suitable challenge dose preliminary experiments were performed. Four doses were evaluated: $5*10^4$, $10^5$, $5*10^5$ and $10^6$ bacteria / ml. In a first experiment using the lab-strain for each dose 10 fish were infected. In a second similar experiment, but this time with isolate 17/Ref0264 five fish per dose were used. These experiments were performed in four individual 38 litre glass aquaria equipped with a flow-through system with tap water and constant aeration. Water temperature was $15.5 \pm 0.5°C$. The fish in the preliminary challenge experiments were fed control diet.

Infection was performed by lowering the water volume to 10 litre and exposing fish to the bacteria for 1 hour. During this time-period water flow was stopped and aeration increased. After the one hour period water flow was re-started and aeration set to normal force.

The daily mortality was then recorded over a period of 14 days.

**Challenge experiment**

After determination of a suitable infection dose fish from each diet group were randomly distributed into three 38 litre tanks per group (20 fish per tank). This resulted in a total of 12 tanks. Then fish were challenged whereby the procedure was the same as for the preliminary experiments. Based on the results of the preliminary experiments the infection dose was set at $3*10^5$ bacteria / ml and isolate 17/Ref0264 was used.

The daily mortality was then recorded, whereby for welfare reasons, moribund fish were removed and euthanized. These animals were counted as died. From the data, mean group values of mortality over time and relative percent survival (RPS) were calculated. For the latter the following formula was used:

1 - (cumulative % mortality of special diet group/cumulative % mortality of control group) $\times$ 100.

The course of the disease was followed 4 weeks until no dead/moribund animals were found for at least 3 consecutive days in any of the tanks.

**Additional investigations**

In the main experiment, first dead fish from each tank were subjected to a bacteriological investigation to confirm the bacteriological aetiology causing death. Bacteria grown from these fish were identified by MALDI-TOFF MS. At the end of the experiment, all surviving fish were assessed for external signs of infection.

**Results**

**Determination of infection dose**

In the preliminary challenge using the laboratory strain no mortality at all occurred indicating that the isolate has lost its pathogenicity to fish. In the second preliminary challenge mortality was obtained in fish challenged with $10^5$, $5*10^5$ and $10^6$ bacteria / ml (table 6). As a mortality rate of 50% in the control group was aimed for, based on the results of the preliminary tests a challenge dose of $3*10^5$ bacteria / ml was chosen.

Table 6: Cumulative mortality of rainbow trout challenged with *Yersinia ruckeri* within 13 days after bath-exposure to bacteria.

| Infection dose | Bacteria / ml of water | | | |
|---|---|---|---|---|
| | $5*10^4$ | $10^5$ | $5*10^5$ | $10^6$ |
| Cumulative mortality % | 0 | 80 | 80 | 40 |

**Challenge experiment**

The first moribund fish were found in a one replicate of the control group 8 days post infection (d.p.i.). On day 10 post infection moribund fish were also reported in tanks of group fed with the composition of the invention. The course of cumulative mortality is shown in Figure 4.

The first moribund fish from every tank was euthanized and analysed for the presence of *Y. ruckeri*. From all investigated fish bacterial cultures could be grown and identification by MALDI-TOFF MS confirmed them to be *Y. ruckeri*. No other bacteria were found. Thus the ERM-pathogen could be determined as the only cause of disease. The moribund animals showed typical features of ERM such as haemorrhagic fins, petechial bleedings in the serosae of the body cavity, haemorrhages in the body musculature and water in the stomach and intestine. No differences in pathology between groups were evident.

Different statistical methods were applied to test the differences between groups for significance; however, differences were not significant between groups.

**Discussion**

Diet supplemented with inactivated Mbb archaea extract (the composition of the invention) had an effect on the course and the cumulative mortality in rainbow trout challenged with the bacterium *Yersinia ruckeri*. The effect was beneficial in fish receiving inactivated Mbb archaea extract as compared to fish receiving a control diet. A positive effect can be attributed to the inactivated archaea extract treatment. Not only was the cumulative mortality lower but also onset of mortality in fish fed inactivated archaea extract was later than in the control.

The finding, that fish fed inactivated archaea extract had lower cumulative mortality than the control group is novel and unexpected.

In summary, a beneficial effect of inactivated Mbb archaea extract on fish challenged with *Yersinia ruckeri* could be demonstrated.

**Claims**

1. A process for preparing a composition comprising attenuated or killed *methanobrevibacter Archaebacteria* cells for incorporation in human food/tablets and/or animal feed/tablets, the process comprising:

   a) collecting a liquid suspension of *methanobrevibacter Archaebacteria* cells;
   b) attenuating or killing said *methanobrevibacter Archaebacteria* cells in said liquid suspension;
   c) freeze drying said liquid suspension to obtain an attenuated or killed *methanobrevibacter Archaebacteria* cell composition, **characterized in that** said attenuated or killed *methanobrevibacter Archaebacteria* cells have preserved at least 80% of the microbe-associated-molecular-patterns (MAMPS) signal potential through the host pattern recognition receptor system, wherein said MAMPS signal potential is determined by the RNA expression level of NF-kB in *in vitro* cell cultures.

2. The process of claim 1, wherein said attenuated or killed *methanobrevibacter Archaebacteria* cell composition is supplemented to a carrier originated from plants or a conventional carrier, **characterized in that** the carrier preferably originated from plants is selected among spirulina, clover, alfalfa, lucerne, oat, wheat, micro-algaes, macro-algaes, barley, corn, soy, rice, cereals, grasses, fruits, vegetables and/or extracts or mixtures thereof.

3. The process according to any of claims 1-2, **characterized in that** attenuating or killing said *methanobrevibacter Archaebacteria* cells in step b) is performed by pasteurization, cryotreatment, infra-red irradiation or air drying techniques.

4. The process according to claim 3, **characterized in that** said pasteurization consists of a short heat pulse treatment performed at a temperature from 50°C to 80 °C for at least 5 to 60 seconds, and wherein said infra-red irradiation is performed at a wave length from 750 nm to 1 millimeter.

5. The process according to any of claims 1-4, wherein the *methanobrevibacter Archaebacteria* cells are *methanobrevibacter smithii.*

6. A composition comprising attenuated or killed *methanobrevibacter Archaebacteria* cells obtained according to the process of any of claims 1-5, **characterized in that** said attenuated or killed *methanobrevibacter Archaebacteria* cells have preserved at least 80% of the microbe-associated-molecular-patterns (MAMPS) signal potential through the host pattern recognition receptor system and wherein said composition comprises at least $10^8$ attenuated or killed *methanobrevibacter Archaebacteria* cells per gram of said composition, for use in a method for improving the resistance to intestinal pathogen infection and/or for reducing intestinal inflammation in a subject.

7. The composition for use according to claim 6, wherein the subject is an animal or a human subject in needs thereof.

8. The composition for use according to claim 7, wherein the animal is a farm animal.

9. The composition for use according to any of claims 7-8, wherein the animal is a bird, mammal or aquatic animal.

10. The composition for use according to any of claims 6-9, wherein the composition is administrated at a dosage of at least $10^9$ attenuated or killed *methanobrevibacter Archaebacteria* cells per kilogram of said subject and per day.

11. The composition for use according to any of claims 6-10, wherein the *methanobrevibacter Archaebacteria* cells are *methanobrevibacter smithii.*

12. The composition for use according to any of claims 6-11, in combination with a vaccination treatment of an animal subject.

13. The composition for use according to any of claims 6-12, wherein the reduction of intestinal inflammation comprises reducing susceptibility to inflammatory bowel disease (IBD) in humans or animal subjects.

14. The composition for use according to any of claims 6-12, wherein the reduction of intestinal inflammation comprises reducing immune pathology and disease severity caused by infections in humans or animal subjects.

15. The composition for use according to any of claims 6-12, wherein the resistance to intestinal pathogen infection comprises promoting innate immune barrier functions and reducing susceptibility to parasitic infections in humans or animal subjects.

**Patentansprüche**

1. Verfahren zur Herstellung einer Zusammensetzung, die abgeschwächte oder abgetötete *Methanobrevibacter-Archaebakterien-Zellen* enthält, zur Einarbeitung in menschliche Nahrungsmittel/Tabletten und/oder Tierfutter/Tabletten, wobei das Verfahren umfasst:

a) das Sammeln einer flüssigen Suspension von *Methanobrevibacter-Archaebakterien-*Zellen;
b) Abschwächen oder Abtöten der besagten *Methanobrevibacter-Archaebakterien-Zellen* in der besagten flüssigen Suspension;
c) Gefriertrocknen der besagten flüssigen Suspension, um eine Zusammensetzung aus abgeschwächten oder abgetöteten *Methanobrevibacter-Archaebakterien-Zellen* zu erhalten, **dadurch gekennzeichnet, dass** die besagten abgeschwächten oder abgetöteten *Methanobrevibacter-Archaebakterien-Zellen* mindestens 80 % des Signalpotenzials der mikrobiell assoziierten molekularen Muster (MAMPS) über das Mustererkennungsrezeptorsystem des Wirts bewahrt haben, wobei das besagte MAMPS-Signalpotenzial durch den RNA-Expressionsgrad von NF-κB in In-vitro-Zellkulturen bestimmt wird.

2. Verfahren nach Anspruch 1, wobei die besagte Zusammensetzung aus abgeschwächten oder abgetöteten *Methanobrevibacter-Archaebakterien-Zellen* einem pflanzlichen Träger oder einem herkömmlichen Träger zugesetzt wird, **dadurch gekennzeichnet, dass** der vorzugsweise pflanzliche Träger ausgewählt ist aus Spirulina, Klee, Alfalfa, Luzerne, Hafer, Weizen, Mikroalgen, Makroalgen, Gerste, Mais, Soja, Reis, Getreide, Gräsern, Obst, Gemüse und/oder Extrakten oder Mischungen davon ausgewählt wird.

3. Verfahren nach einem beliebigen der Ansprüche 1-2, **dadurch gekennzeichnet, dass** das Abschwächen oder Abtöten der besagten *Methanobrevibacter-Archaebakterien-Zellen* in Schritt b) durch Pasteurisierung, Kryobehandlung, Infrarotstrahlung oder Lufttrocknungstechniken erfolgt.

4. Verfahren nach Anspruch 3, **dadurch gekennzeichnet, dass** die besagte Pasteurisierung aus einer kurzen Hitzepulsbehandlung besteht, die bei einer Temperatur von 50 °C bis 80 °C für mindestens 5 bis 60 Sekunden durchgeführt wird, und wobei die besagte Infrarotstrahlung bei einer Wellenlänge von 750 nm bis 1 Millimeter durchgeführt wird.

5. Verfahren nach einem beliebigen der Ansprüche 1 bis 4, wobei die *Methanobrevibacter*-Archaebakterien-Zellen *Methanobrevibacter smithii* sind.

6. Eine Zusammensetzung, die attenuierte oder abgetötete *Methanobrevibacter*-Archaebakterien-Zellen umfasst, die gemäß dem Verfahren nach einem beliebigen der Ansprüche 1 bis 5 erhalten wurden, **dadurch gekennzeichnet, dass** die besagten attenuierten oder abgetöteten *Methanobrevibacter-Archaebakterien-Zellen* mindestens 80 % des Signalpotenzials der mikrobienassoziierten molekularen Muster (MAMPS) über das Mustererkennungsrezeptorsystem des Wirts bewahrt haben, und wobei die besagte Zusammensetzung mindestens $10^8$ abgeschwächte oder abgetötete *Methanobrevibacter*-Archaebakterien-Zellen pro Gramm der besagten Zusammensetzung enthält, zur Verwendung in einem Verfahren zur Verbesserung der Resistenz gegen eine Infektion mit Darmpathogenen und/oder zur Verringerung einer Darmentzündung bei einem Patienten.

7. Die Zusammensetzung zur Verwendung gemäß Anspruch 6, wobei der Patient ein Tier oder ein Mensch ist, der eine solche Behandlung benötigt.

8. Zusammensetzung zur Verwendung gemäß Anspruch 7, wobei das Tier ein Nutztier ist.

9. Zusammensetzung zur Verwendung gemäß einem beliebigen der Ansprüche 7 bis 8, wobei das Tier ein Vogel, ein Säugetier oder ein Wassertier ist.

10. Die Zusammensetzung zur Verwendung gemäß einem beliebigen der Ansprüche 6 bis 9, wobei die Zusammensetzung in einer Dosierung von mindestens $10^9$ abgeschwächten oder abgetöteten *Methanobrevibacter-Archaebakterien-Zellen* pro Kilogramm des besagten Patienten und pro Tag verabreicht wird.

11. Die Zusammensetzung zur Verwendung gemäß einem beliebigen der Ansprüche 6 bis 10, wobei die *Methanobrevibacter-Archaebakterien-Zellen Methanobrevibacter smithii* sind.

12. Die Zusammensetzung zur Verwendung gemäß einem beliebigen der Ansprüche 6 bis 11, in Kombination mit einer Impfbehandlung eines Tierpatienten.

13. Die Zusammensetzung zur Verwendung gemäß einem beliebigen der Ansprüche 6 bis 12, wobei die Verringerung der Darmentzündung die Verringerung der Anfälligkeit für entzündliche Darmerkrankungen (IBD) bei Menschen oder Tierpatienten umfasst.

14. Die Zusammensetzung zur Verwendung gemäß einem beliebigen der Ansprüche 6 bis 12, wobei die Verringerung der Darmentzündung die Verringerung der durch Infektionen verursachten Immunpathologie und des Schweregrads der Erkrankung bei Menschen oder Tierpatienten umfasst.

15. Die Zusammensetzung zur Verwendung gemäß einem beliebigen der Ansprüche 6 bis 12, wobei die Resistenz gegen eine Infektion mit Darmpathogenen die Förderung angeborener Immunbarrierefunktionen und die Verringerung der Anfälligkeit für parasitäre Infektionen bei Menschen oder Tierpatienten umfasst.

**Revendications**

1.  Procédé de préparation d'une composition comprenant des cellules *d'archéobactéries Methanobrevibacter* atténuées ou tuées, destinée à être incorporée dans des aliments/comprimés pour humains et/ou des aliments/comprimés pour animaux, le procédé comprenant :

    a) la collecte d'une suspension liquide de cellules *d'archéobactéries Methanobrevibacter ;*
    b) l'atténuation ou la destruction desdites cellules *d'archéobactéries Methanobrevibacter* dans ladite suspension liquide ;
    c) la lyophilisation de ladite suspension liquide pour obtenir une composition de cellules *d'archéobactéries Methanobrevibacter* atténuées ou tuées, **caractérisé en ce que** lesdites cellules *d'archéobactéries Methanobrevibacter* atténuées ou tuées ont conservé au moins 80 % du potentiel de signal des motifs moléculaires associés aux microbes (MAMPS) par le biais du système de récepteurs de reconnaissance des motifs de l'hôte, ledit potentiel de signal MAMPS étant déterminé par le niveau d'expression de l'ARN de NF-kB dans des cultures cellulaires *in vitro.*

2.  Procédé selon la revendication 1, dans lequel ladite composition de cellules *d'archéobactéries Methanobrevibacter* atténuées ou tuées est ajoutée à un support d'origine végétale ou à un support conventionnel, **caractérisée en ce que** le support d'origine végétale est de préférence choisi parmi la spiruline, le trèfle, la luzerne, l'avoine, le blé, les microalgues, les macroalgues, l'orge, le maïs, le soja, le riz, les céréales, les graminées, les fruits, les légumes et/ou les extraits ou des mélanges de ceux-ci.

3.  Procédé selon l'une quelconque des revendications 1 à 2, **caractérisé en ce que** l'atténuation ou la destruction desdites cellules *d'archéobactéries Methanobrevibacter à* l'étape b) est réalisée par des techniques de pasteurisation, de cryotraitement, d'irradiation infrarouge ou de séchage à l'air.

4.  Procédé selon la revendication 3, **caractérisé en ce que** ladite pasteurisation consiste en un traitement par impulsion thermique de courte durée effectué à une température comprise entre 50 °C et 80 °C pendant au moins 5 à 60 secondes, et dans lequel ladite irradiation infrarouge est effectuée à une longueur d'onde comprise entre 750 nm et 1 millimètre.

5.  Procédé selon l'une quelconque des revendications 1 à 4, dans lequel les cellules *d'archéobactéries Methanobrevibacter* sont des *Methanobrevibacter smithii.*

6.  Composition comprenant des cellules *d'archéobactéries Methanobrevibacter* atténuées ou tuées obtenues selon le procédé de l'une quelconque des revendications 1 à 5, **caractérisée en ce que** lesdites cellules *d'archéobactéries Methanobrevibacter* atténuées ou tuées ont conservé au moins 80 % du potentiel de signal des motifs moléculaires associés aux microbes (MAMPS) par l'intermédiaire du système de récepteurs de reconnaissance des motifs de l'hôte, et dans laquelle ladite composition comprend au moins $10^8$ cellules *d'archéobactéries Methanobrevibacter* atténuées ou tuées par gramme de ladite composition, pour l'utilisation dans une méthode visant à améliorer la résistance à une infection par des agents pathogènes intestinaux et/ou à réduire l'inflammation intestinale chez un sujet.

7.  Composition pour l'utilisation selon la revendication 6, dans laquelle le sujet est un animal ou un sujet humain qui a besoin d'un tel traitement.

8.  Composition pour l'utilisation selon la revendication 7, dans laquelle l'animal est un animal d'élevage.

9.  Composition pour l'utilisation selon l'une quelconque des revendications 7 à 8, dans laquelle l'animal est un oiseau, un mammifère ou un animal aquatique.

10. Composition pour l'utilisation selon l'une quelconque des revendications 6 à 9, dans laquelle la composition est administrée à une dose d'au moins $10^9$ cellules *d'archéobactéries Methanobrevibacter* atténuées ou tuées par kilogramme dudit sujet et par jour.

**11.** Composition pour l'utilisation selon l'une quelconque des revendications 6 à 10, dans laquelle les cellules *d'archéobactéries Methanobrevibacter* sont des *Methanobrevibacter smithii.*

**12.** Composition pour l'utilisation selon l'une quelconque des revendications 6 à 11, en association avec un traitement vaccinal d'un sujet animal.

**13.** Composition pour l'utilisation selon l'une quelconque des revendications 6 à 12, dans laquelle la réduction de l'inflammation intestinale comprend la réduction de la susceptibilité aux maladies inflammatoires chroniques de l'intestin (MICI) chez les sujets humains ou animaux.

**14.** Composition pour l'utilisation selon l'une quelconque des revendications 6 à 12, dans laquelle la réduction de l'inflammation intestinale comprend la réduction de la pathologie immunitaire et de la gravité de la maladie causées par des infections chez l'homme ou chez des sujets animaux.

**15.** Composition pour l'utilisation selon l'une quelconque des revendications 6 à 12, dans laquelle la résistance à l'infection par des agents pathogènes intestinaux comprend la promotion des fonctions de barrière immunitaire innée et la réduction de la susceptibilité aux infections parasitaires chez l'homme ou chez des sujets animaux.

Fig. 1 (a)

**Fig. 1 (b)**

**Fig. 1 (c)**

**Fig. 2**

1

4            6

2

5            7

3

**Fig. 3 (A) & (B)**

**Fig. 3 (C) & (D)**

Cryo treatment (C)

72C 15' treatment (D)

Fig. 3 (E)

**Fig. 4**

Cumulative mortality in fish infected with *Yersinia ruckeri*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016147121 A1
- EP 2251017 A1
- US 6328959 B1, Kayar S.
- US 2012034198 A1, Garner
- US 2016074440 A1, Brugere

**Non-patent literature cited in the description**

- **WHITMAN et al.** The Prokaryotes. Springer Verlag, 2014, 123-163
- **CORINNA BANG et al.** The Intestinal Archaea Methanosphaera stadtmanae and Methanobrevibacter smithii Activate Human Dendritic Cells. *PLOS ONE*, 10 June 2014, vol. 9 (6), E99411
- **UDAY C GHOSHAL et al.** Slow Transit Constipation Associated with Excess Methane Production and Its Improvement Following Rifaximin Therapy: A Case Report''. *J. Neurogastroenterol Motil*, 27 April 2011, https://www.ncbi.nlm.nih.gov/pmc/articles/PMC3093012/
- **ATTALURI A. et al.** Methanogenic flora is associated with altered colonic transit but not stool characteristics in constipation without IBS. *Am. J. Gastroenterol.*, June 2010, vol. 105 (6), 1407-11, https://www.ncbi.nlm.nih.gov/pubmed/19953090
- **TRINE H. MOGENSEN**. Pathogen Recognition and Inflammatory Signalling in Innate Immune Defenses. *Clin Microbiol Rev.*, April 2009, vol. 22 (2), 240-273, https://www.ncbi.nlm.nih.gov/pmc/articles/PMC2668232/